# EUROPEAN PATENT APPLICATION

(11) **EP 2 383 339 A1**
(43) Date of publication of application: **02.11.2011**
(21) Application number: 10161220.8
(22) Date of filing: 27.04.2010
(51) Int. Cl.: C12N 15/10, A61K 48/00

(54) **Gene trapping for use in gene therapy**

(71) Applicant: Von Melchner, Harald, 60590 Frankfurt am Main (DE); Grez, Manuel, 60596 Frankfurt (DE)
(72) Inventor: Grez, Manuel, 69121, Heidelberg (DE); Schnütgen, Frank, 63755, Alzenau (DE); Von Melchner, Harald, 61474, Kronberg (DE); Schultze-Strasser, Stephan, 63477, Maintal (DE)
(74) Representative: von Kreisler Selting Werner

(57) **Abstract**

The present invention provides gene trap constructs for use in gene therapy, notably gene trap constructs for the correction of the Gp91phox (Nox2) mutation in chronic granulomatous disease (X-CGD).

## Description

The present invention provides gene trap constructs for use in gene therapy, notably gene trap constructs for the correction of the Gp91phox (Nox2) mutation in chronic granulomatous disease (X-CGD).

### Background of the Invention

Retroviruses are highly efficient insertional mutagens that have been used successfully to isolate and characterize a multitude of cancer genes from various cells and organisms. Due to their unique ability to activate cellular oncogenes from promoter proximal insertions, retroviruses are among the most effective genetic tools to study oncogenic signaling and oncogene cooperation. For example, a recent high throughput screen of over 10,000 retroviral insertion sites in mice identified over 300 genes implicated directly or indirectly in tumorigenesis Uren AG et al., Cell 133, 727-741 (2008).

In addition to insertional mutagenesis, retroviruses are widely used to stably express genes of interest in mammalian cells for functional analysis. Based on their ability to permanently insert expression cassettes into the genome, retroviruses are also preferred gene delivery vehicles in gene therapy approaches aimed at correcting monogenic defects in somatic cells, particularly in hematopoietic cells. Numerous preclinical animal studies have shown that the *ex vivo* transduction of genes into hematopoietic stem and progenitor cells (HSC) followed by bone marrow transplantation results in long lasting expression of the transgenes at levels high enough to permanently correct various monogenic blood defects (reviewed in Klein C & Baum C, Hematol J 5, 103-111 (2004)). Moreover, several clinical trials provided definite proof for the curative potential of retroviral gene therapy of otherwise intractable blood disorders. However, this cure carried a price: several patients from different trials developed myeloproliferative disorders and leukemias (reviewed in Kaiser J, Science New York, NY 308, 1735-1736 (2005); Ferguson C et al., Trends in biotechnology 23, 589-597 (2005); Baum C et al., Human gene therapy 17, 253-263 (2006); Biffi A & Cesani M, Current gene therapy 8, 135-146 (2008); Daniel R & Smith JA, Human gene therapy 19, 557-568 (2008)).

Thanks to the availability of the mouse and human genome sequences and by the development of high throughput strategies for the recovery of retroviral insertion sites from mammalian genomes Uren AG et al., Cell 133, 727-741 (2008); Harkey MA et al., Stem cells and development 16, 381-392 (2007); Horn C et al., Nat Genet 39, 807-808 (2007), it is presently well established that retroviruses and retroviral vectors alike do not integrate randomly throughout the genome. Genome wide studies in mammalian cells demonstrated that retroviruses have a high integration preference near the transcriptional start sites of expressed genes Bushman FD, Cell 115, 135-138 (2003); Wu X et al., Science (New York, NY 300, 1749-1751 (2003); Bushman F et al., Nature reviews 3, 848-858 (2005). As a result, the proviral promoter/enhancer elements driving the expression of a therapeutic gene can simultaneously activate the expression of adjacent genes. Since most insertions are promoter proximal or are in the most 5' introns of genes, the induced transcripts frequently encode full length and fully functional cellular proteins. Thus, their overexpression can cause gain of function mutations that lead to aberrant cellular phenotypes. It is exactly this ability of retroviruses to induce dominant mutations that has been and is being exploited in phenotype driven screens for transforming genes by using clonal expansion and tumorigenesis as convenient phenotypic markers (reviewed in Uren AG et al., Oncogene 24, 7656-7672 (2005)).

The ability of retroviruses to stably transduce and express therapeutic genes in hematopoietic cells is thus offset by the high risk of cancer imposed by activated oncogenes. It is presently unclear to what extent the retroviral activation of non-transforming genes affects normal cell physiology. Since insertions have been identified in most functional gene classes defined by GeneOntology Cornils K et al., Mol Ther 17, 131-143 (2009); Schnutgen F et al., Nucleic acids research 36, e133 (2008), one must assume that genes controlling functions other than cell growth are also subject to mutational activation and could thus affect the therapeutic outcome. For example, the frequently observed repression of transgene expression by DNA methylation of retrovirally transduced hematopoietic cells in the transplanted hosts may well involve mutations of DNA-methylase encoding genes. Overall, past and present gene therapy experience suggests that retroviral vectors lacking endogenous gene activating elements are highly desirable.
Several strategies have been employed to reduce the oncogenic risk inflicted by retroviral vectors including (i) the relocation of the U3 promoter/enhancer elements into the viral body between the long terminal repeats (LTRs) (self inactivating [SIN] vectors) to prevent transcriptional initiation or enhancement by the 3'LTR Schambach A et al., Mol Ther 13, 391-400 (2006), (ii) the replacement of viral promoter elements with cellular promoters and tissues specific enhancers to restrict gene expression to differentiated cells Zychlinski D et al., Mol Ther 16, 718-725 (2008), (iii) the use of chromosomal insulator elements to shield the flanking DNA from the exogenous transcription units Ramezani A et al., Stem cells (Dayton, Ohio) 26, 3257-3266 (2008), and (iv) the use of self inactivating lentiviral backbones which unlike retroviruses have no promoter proximal integration preference Schroder AR et al., Cell 110, 521-529 (2002); De Palma M et al., Blood 105, 2307-2315 (2005).

While each of the above designs reduced the frequency of oncogenic mutations when rigorously tested in dedicated leukemogenicity assays, none of them eliminated the risk completely (reviewed in Daniel R & Smith JA, Human gene therapy 19, 557-568 (2008); Schambach A & Baum C, Current gene therapy 8, 474-482 (2008)). The safest vector tested thus far was an enhanced green fluorescent protein (EGFP) transducing retrovirus reduced completely to its backbone. Devoid of any gene regulatory elements, this vector was incapable of inducing clonal outgrowth in bone marrow transplanted mice. Unfortunately, the level of EGFP transgene expression from this vector was far below the level considered adequate for the functional correction of a genetic defect Cornils K et al., Mol Ther 17, 131-143 (2009).

One way of improving gene expression from these vectors lacking regulatory elements would be to link the transduced therapy gene to the promoter/enhancer elements of an endogenous gene by using a gene trapping approach (reviewed in Nord AS et al., Nucleic acids research 34, D642-648 (2006)).

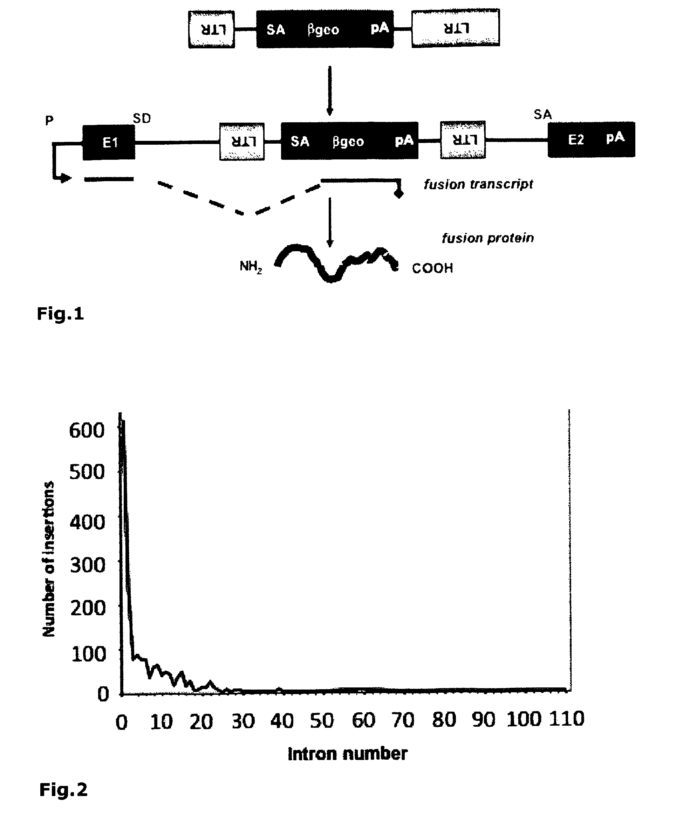

Special SIN retroviral vectors known as gene traps have been developed for the high throughput production of mutant embryonic stem (ES) cell lines that can be used to make knock-out mice Wiles MV et al., Nat Genet 24, 13-14 (2000); Hansen J et al., Proc Natl Acad Sci U S A 100, 9918-9922 (2003); Skarnes WC et al., Nat Genet 36, 543-544 (2004); Hansen GM et al., Genome research 18, 1670-1679 (2008). The principal elements of the most widely used gene traps are a promoterless reporter and/or selectable marker gene flanked by an upstream 3' splice site (splice acceptor; SA) and a downstream transcriptional termination sequence (polyadenylation sequence; polyA) (Figure 1). When inserted into an intron of an expressed gene, gene traps are transcribed from an endogenous promoter, yielding fusion transcripts in which the upstream exons are spliced to the reporter/selectable marker gene. Since transcription is terminated prematurely at the inserted polyadenylation site, the processed fusion transcript encodes a truncated and nonfunctional version of the cellular protein plus the reporter/selectable marker, or - if the insertion is upstream of initiating methionines - just encodes the reporter selectable/marker. Unlike the dominant gain of function mutations induced by the classic retroviral vectors, most gene trap mutations are recessive and require conversion to homozygosity for functional analysis. In mice, this is typically achieved by breeding the mutations to homozygosity.

Unlike classic retroviruses and retrovirus vectors, gene traps are incapable of activating transforming genes and with a few exceptions such as insertions into single copy X,Y chromosome linked or haploinsufficient genes, heterozygous gene trap mutations will not cause a phenotype because most mammalian genes are recessive, including most tumor suppressor genes. Moreover, even loss of function mutations in the few tumor suppressor genes for which tissue specific haploinsufficiency has been described (reviewed in Payne SR & Kemp CJ, Carcinogenesis 26, 2031-2045 (2005)) are unlikely to be transforming without a cooperating oncogene. In this context, it should be noted that the risk of developing leukemia in patients receiving autologous bone marrow transplants for non-hematologic malignancies is extremely low, suggesting that activating oncogene mutations in transplanted hematopoietic cells are rare (reviewed in Hake CR et al., Bone marrow transplantation 39, 59-70 (2007)). Therefore, we believe that a gene trap gene therapy approach in hematopoietic cells will be safer and perhaps even more effective than the standard methodology.

### Summary of the Invention

Retroviruses are widely used to transduce and stably express genes of interest in hematopoietic stem cells (HSC). Several clinical trials employing retroviruses for the *ex vivo* transduction of healthy genes into hematopoietic stem cells from patients with monogenic blood disorders provided definite proof for curative potential of gene therapy. However, these trials also showed that retroviral gene therapy can cause leukemia by the insertional activation of cellular oncogenes. Despite multiple efforts to circumvent these oncogenic side effects, only the deletion of all gene regulatory elements from retroviral vectors completely eliminated the risk of clonal expansion. However, such vectors are poor gene expressors and hence inadequate for therapeutic purposes. To address this issue, a gene trapping approach was found, which enables expression of the transduced gene from cellular promoters. This gene trapping approach using low risk retroviral vectors will enable a safe and effective transduction of therapeutic genes into hematopoietic stem cells. The invention thus provides
(1) a gene trap vector for use in gene therapy, which comprises a target gene cassette (hereinafter shortly referred to as "TGC") and one or more enhancer elements, wherein said TGC comprises a promoterless target gene flanked by an upstream 3' splice acceptor (SA) site and a downstream transcriptional termination (polyA) sequence;
(2) a gene trap vector, which comprises a target gene cassette (TGC) and one or more enhancer elements, wherein said TGC comprises a promoterless target gene, which is a nucleic acid sequence for correcting a genetic defect of a patient, flanked by an upstream 3' splice acceptor (SA) site and a downstream transcriptional termination (polyA) sequence;
(3) the use of a gene trap vector as defined in (1) or (2) above for preparing a medicament for performing gene therapy; and
(4) a method for gene therapy comprising administering a patient in need of a gene therapy a gene trap vector as defined in (1) above.

### Short Description of the Figures

Figure 1: Classic retroviral gene trap vector and mechanism of activation. See text for further explanation. LTR, long terminal repeat; SA, splice acceptor site; βgeo, β-galactosidase-neomycinphosphotransferase fusion gene; pA, polyadenylation site; P, cellular promoter; E, exon; SD, splice donor.
Figure 2: Integration preference of retroviral gene trap vectors into the 5' introns of annotated ENSEMBL genes.

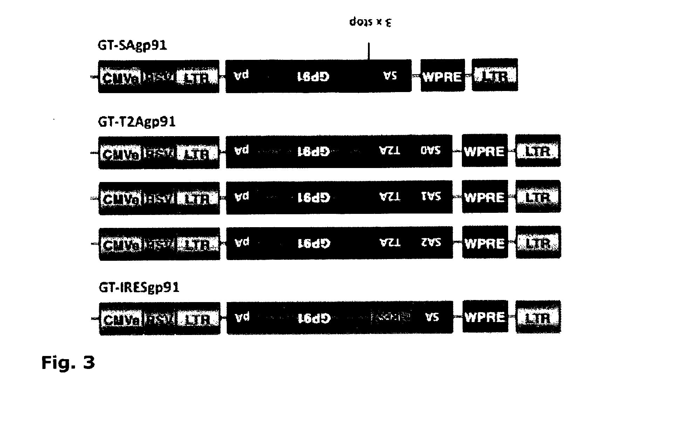

Figure 3: Schematic representation of the GP91phox gene trap vectors GT-SAgp91, GT-T2Agp91, GT-EDN-SAgp91 and GT-EDN-SAgp91 (SEQ ID NOs:1-4). RSV, Rous sarcoma virus promoter; LTR, long terminal repeat; pA, bovine growth hormone polyadenylation sequence; SA, type II adenovirus splice acceptor sequence; T2A, polyprotein cleavage sequence; EDN, eosinophil-derived neurotoxin enhancer; WPRE, woodchuck hepatitis virus posttranscriptional regulatory element; IRES, EMCV internal ribosomal entry site.

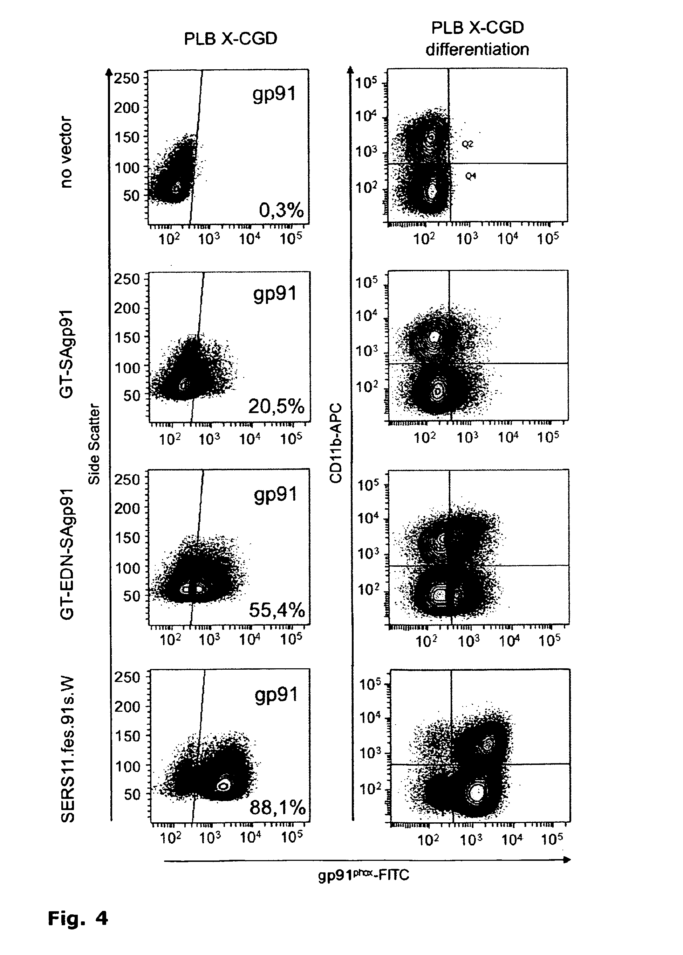

Figure 4: Transduction of PLB X-CGD cells with GT-EDN-SA-gp91 gene trap virus. A SIN gammaretroviral vector expressing gp91phox from an internal c-fes promoter was used as a control. The fraction of transduced cells was measured by flow cytometry using the monoclonal 7D5 anti-human gp91 antibody before (left panel) and after (right panel) granulocytic differentiation induced by dimethylsulfoxide (DMSO).

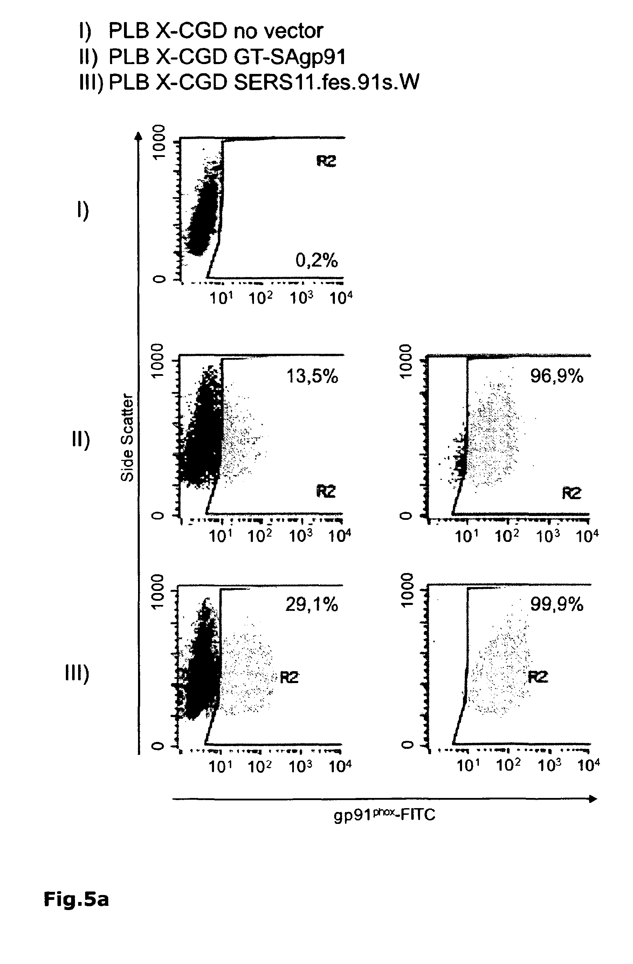

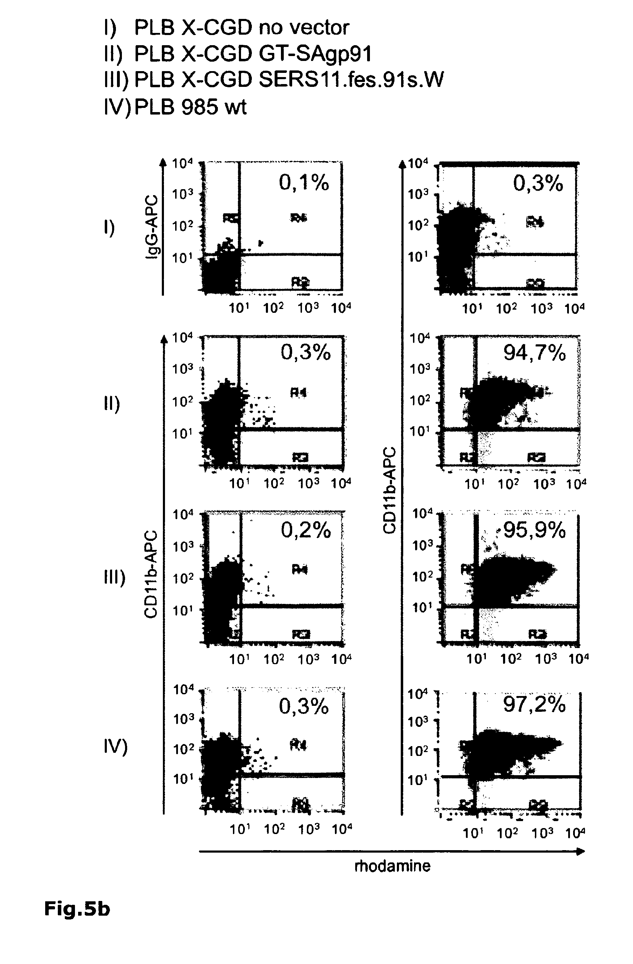

Figure 5: Functional reconstitution of superoxide production in PLB X-CGD cells transduced with the gene trap vector GT-SAgp91. A) gp91phox expression measured by FACS. Left panels: starting population. Right panels: MACS sorted population after induction of differentiation with DMSO. B. Superoxide production in FACS sorted gp91phox positive cells. Cells were exposed to DMSO for 6 days and incubated with with DHR-123. Rhodamine fluorescence is shown for cells before (A) and after (B) stimulation with PMA. PLB X-CGD, Nox2 knock out PLB985 cells (negative control); PLB985, wild type myelomonocytic cells (positive control).

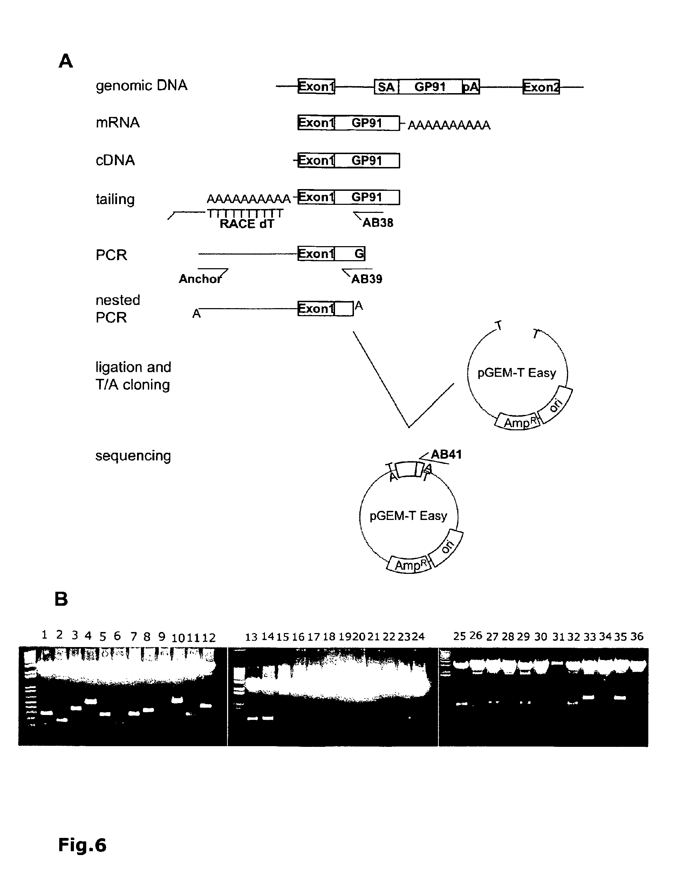

Figure 6: Characterization of GT-SAgp91 insertion sites. A: 5'RACE strategy and shot gun cloning. B: Ethidium bromide stained agarose gels showing examples of cloned 5'RACE products.

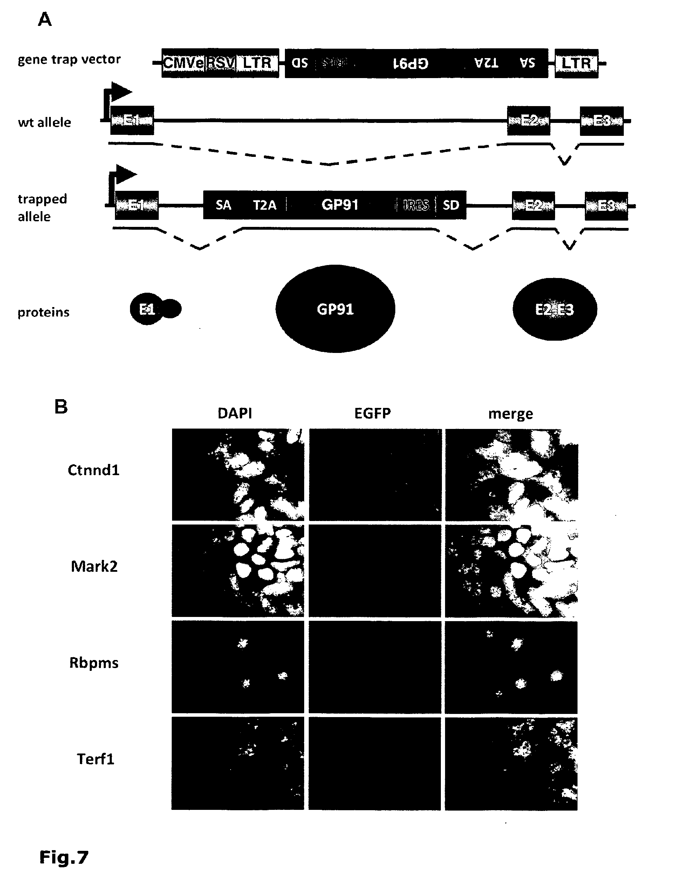

Figure 7: SAT2Agp91SD gene trap vector (SEQ ID NO:5). A: Schematic representation and mechanism of activation. B: Examples of eGFP tagged proteins by combined SA/SD gene trap vectors and their subcellular localization in ES cells. eGFP-tagged proteins were visualized by indirect immunofluorescence. Cells were grown on cover slips for two days, stained with a polyclonal anti-egfp antibody, counterstained with DAPI and analyzed using a standard fluorescence microscope at × 100 magnification.

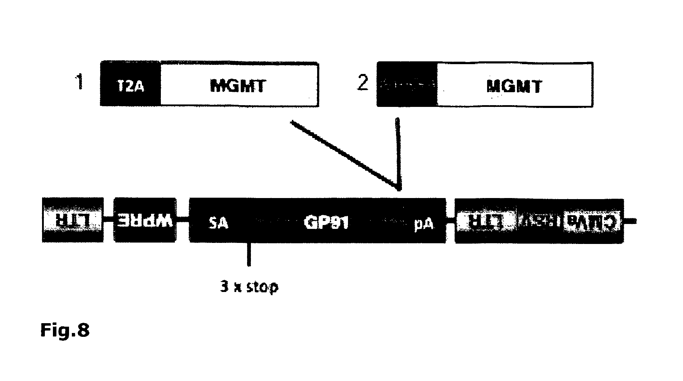

Figure 8: Design of the GT-SAgp91-MGMT vectors for in vivo selection.

### Detailed Description of the Invention

The gene trap vector for use in gene therapy of aspect (1) of the invention (hereinafter also shortly referred to as "gene trap vector of the invention") comprises a TGC and one or more enhancer elements, wherein said TGC comprises a promoterless target gene flanked by an upstream 3' splice acceptor (SA) site and a downstream transcriptional termination (polyA) sequence.

The "target gene" within the meaning of the invention may be any nucleic acid sequence that is capable of correcting a genetic defect of a patient. In aspects (1) and (2) of the invention such target gene may be an intact gene for expression in a patient with a genetic loss of function mutation, including, but not limited to, a gene encoding coagulation factor VIII, coagulation factor IX, adenosine deaminase, common immunoglobulin gamma chain, adrenoleukodystrophy protein, hemoglobin etc. It is particularly preferred that the target gene encodes a gp91phox, notably the gp91phox encoded by the nucleic acid sequence of 3200-4912 of SEQ ID NO:1. Alternatively, the target gene may be a gene inactivating sequence for the knock out of a gene expression in a patient, preferably encoding an interfering short hairpin (shRNA) or micro RNA (miRNA) or an antisense transcript.

In said gene trap vector of aspects (1) and (2) of the invention t is preferred that said one or more enhancer elements are tissue specific enhancer elements which may be located (i) upstream of the TGC, or (ii) downstream of the TGC.

Furthermore, the target gene may be fused in frame to an upstream polyprotein cleavage sequence. Such cleavage sequences include, but are not limited to, P2A and T2A from Picorna virus.

Also the target gene may be fused to an upstream internal ribosomal entry site (IRES)

In another preferred embodiment of aspect (1) of the invention the polyA sequence of the TGC is replaced by a 5'splice donor site.

In the gene trap vector of the invention, the TGC and the one or more enhancer elements may be contained in a genome integrating plasmid or virus, preferably they are contained in a self-inactivating lentivirus, most preferably in a self-inactivating retrovirus.

In a preferred embodiment the one or more enhancer elements contain at least one binding site for a transcription activating factor.

In a further preferred embodiment the one or more enhancer elements contain binding sites that bind transcription activation factors in a sequence-specific manner. It is particularly preferred that the binding sites are arranged as tandem repeats.

In a further preferred embodiment the one or more enhancer elements comprise tissue specific transcription factor binding elements. Particularly preferred binding elements include, but are not limited to, the c-fms regulatory intronic element (FIRE) from the second intron of the CSF1-receptor encoding gene, the CD68 enhancer from the first intron of the gene encoding microsialin, etc. Here the Eosinophil-Derived Neurotoxin (EDN) enhancer from the first intron of the EDN encoding gene is most preferred.

Still in a further preferred embodiment the one or more enhancer elements are tandem repeats of AP-1, AP-2, CRE, SRE, NF-kB, SRF, SP1, Oct1, Oct2, Oct3, Oct4 transcription factor binding sites. The particularly preferred enhancer element is an Oct-4 responsive enhancer element.

The gene trap vector of the invention may further comprise a selectable marker and/or a reporter gene. Such selectable marker includes, but is not limited to, the P140K-O(6)-methylguanine-methyltransferase (MGMT/P140K) gene, the puromycin resistance gene, the neomycinphosphotransferase fusion gene, the hygromycin resistance gene and the HSV thymidine kinase gene. A particularly preferred selectable marker is the MGMT/P140K gene. The reporter gene includes, but is not limited to, a β-galactosidase gene, the enhanced greeen fluorescence protein (egfp) and the luciferase gene.

It is preferred in the gene trap vector of the invention that the selectable marker or reporter gene is inserted into the TGC upstream or downstream of the target gene using a 5' IRES or a polyprotein cleavage sequence.

The gene trap vector of the invention is particularly be used in gene therapy for the correction of chronic granulomatous disease, where it has preferably the structure depicted in Fig. 3 or 7 and has the sequence shown in SEQ ID NOs:1 to 5. Concerning the preferred embodiments of aspects (3) and (4) of the invention it is referred to the discussion of aspect (1) above.

A patient within the meaning of the invention may be any individual in need of a gene therapy including human beings and animals.

It was recently shown that the insertion of the Oct4 transcription factor binding osteopontin enhancer (OPE) into a gene trap vector increased the efficiency of trapping in ES cells by 25% by directly activating the captured endogenous promoter Schnutgen F et al., Nucleic acids research 36, e133 (2008). Based on these results it is anticipated that the use of intronic enhancer elements responsive to transcription factors differentially expressed in the cells that require genetic correction would also enhance the expression of a therapeutic gene from the captured cellular promoters. For example, in the context of X-CGD, the following enhancer elements active in hematopoietic cells are suitable for the gene trap vectors of the invention, notably the gp91 gene trap vectors: (i) c-fms regulatory intronic element (FIRE) from the second intron of the CSF1-receptor encoding gene Sasmono RT et al., Blood 101, 1155-1163 (2003), (ii) Eosinophil-derived neurotoxin (EDN) enhancer from the first intron of the EDN encoding gene van Dijk TB et al., Blood 91, 2126-2132 (1998) and (iii) the CD68 enhancer from the first intron of the gene encoding microsialin Gough PJ & Raines EW, Blood 101, 485-491 (2003). All three enhancers are responsive to the myeloid specific transcription factor PU.1. Figure 4 shows that the insertion of the EDN enhancer 5'to the splice acceptor site of the GT-SAgp91 gene trap vector significantly increased the gp91 transduction into Nox2 deficient human hematopoietic (X-CGD PLB985) cells..

A second class of gene trap gene therapy aims at minimizing the risk of single copy- or haploinsufficient gene inactivation. As shown in Figure 7A, these vectors contain in addition to a SAT2Agp91 cassette a downstream IRES linked to a splice donor (SD) sequence. When integrated into a 5'intron of an expressed gene, the therapeutic gene, e.g. gp91 is translated as a native protein from a fusion transcript as described in Section 3.2. Due to the SD sequence, gp91IRES is spliced to the downstream exons of the trapped gene (Figure 7A). Since in about 40% of all genes first exons are non-coding and most of the first coding exons are very short, the majority of the gene trap insertions will enable the expression of a full-or almost full length endogenous transcript. In insertions downstream of non-coding exons the IRES will insure translational initiation at the endogenous AUG. However, insertions downstream of coding exons will require an initiating AUG for endogenous protein expression. To achieve this, the IRES upstream of the SD site of the said gene trap vector contains ATGs in all translational reading frames. This was previously shown to enable robust translation of exon sequences residing downstream of various gene trap integration sites Shigeoka T et al., Nucleic acids research 33, e20 (2005). More recently, we have used a similar splice donor gene trap vector to attach an N-terminal eGFP tag to proteins expressed in embryonic stem cells. Figure 7B shows some examples of successful in situ protein tagging by using this strategy.

All gene trap vectors are evaluated, notably in the X-CGD PLB985 cell line as described in Example 3. For the best performing vectors, several hundred gp91 expressing clones are isolated by single cell sorting to determine the number and location of gene trap insertions sites using Southern blotting and a combination of high throughput 5'RACE and splinkerette PCR. PCR products are directly sequenced and mapped to the genome using the ENSEMBL /NCBI databases (also see Example 4). The ultimate goal is to obtain polyclonal cell populations with single copy gene trap insertions expressing a fully functional therapy gene such as gp91.

The best performing vectors are used to transduce lineage negative (Lin-) murine X-CGD bone marrow cells. Gp91 expressing cells are sorted either immunomagnetically or by FACS, and tested after granulocytic differentiation for functional activity in DHR assays. Also the entire population is subjected to high throughput 5'RACE and/or splinkerette PCR followed by shot gun cloning. Cloned inserts are sequenced and mapped to the genome as described above.

Long term reconstitution of superoxide production and persistence of gene expression in X-CGD mice transplanted with gene trap modified cells are assessed. Lineage negative (Lin-) bone marrow (BM) cells from male X-CGD mice are isolated and transduced with a gp91 gene trap vector and a control SIN vector expressing gp91 from an internal promoter at multiplicities of infection (MOIs) of 3 to 10 according to our standard protocols. Transgene expression is monitored by flow cytometry and qPCR to control for equal transduction efficiencies in all cultures. After transduction, cells are injected into lethally irradiated female recipients and the animals are examined every 3 weeks for functional gp91 expression and hematopoietic lineage differentiation using FACS and functional NADPH oxidase assays (DHR). Moreover, copy number and integration site analysis is to be estimated at the end of the observation period (month 8). Subsequently secondary transplantations are performed to assess gene transfer efficiency into long term repopulating cells as well as long-lasting gp91phox expression. The secondary recipients are observed and analyzed as described above for an additional 3 months.

If the transduction efficiency in Lin- cells is below 10%, a selectable marker gene is to be included to expand the transduced cells *in vivo.* The P140K-O(6)-methylguanine-methyltransferase (MGMT/P140K) gene has been widely used for this purpose. MGMT/P140K mediated expansion of transduced hematopoietic cells is achieved by treating sublethally irradiated recipients with O(6) alkylating agents combined with endogenous MGMT inhibitors, a drug combination shown to retain the engraftment ability of the expanded cells (reviewed in Zaboikin M et al., Cancer gene therapy 13, 335-345 (2006); Schambach A & Baum C, DNA repair 6, 1187-1196 (2007); Milsom MD & Williams DA, DNA repair 6, 1210-1221 (2007)).

Thus, the coding sequence of MGMT(P140K) is inserted into the GT-SAgp91 vector downstream of the gp91phox cDNA whereby the two cDNAs are linked by using either a T2A element or n IRES in case gp91 does not tolerate C-terminal fusions (Figure 7). GT-SAgp91MGMT transduced murine Lin- cells are transplanted into sublethally irradiated recipients, which receive a drug combination consisting of an O(6)-alkylating agent (e.g., temozolomide) and an endogenous MGMT inhibitor (e.g O(6)-benzylguanine) at weeks 10, 15 and 20 after transplantation. Following selection, animals are observed and processed as described above.

A safety analysis for detecting the effects of gene trap vector integration on cell proliferation, differentiation and transformation is performed. Overall, this analysis measures the potential of a vector to induce growth promoting gene mutations. First an *in vitro* toxicity assay developed by Modlich et al Modlich U, Blood 108, 2545-2553 (2006) is implemented, in which Lin- bone marrow cells are transduced at different MOIs and expanded in mass culture for two weeks. Following expansion cells are plated onto 96 well plates at a density of 10 or 100 cells/well. After 14 days, wells showing cell proliferation are counted and the replating efficiency is calculated using Poisson statistics. Finally, selected clones are expanded for copy number and integration site analysis. Appropriate positive (SFFV-eGFP) control vectors are used to validate the assay.

For the *in vivo* experiments, Lin- from C57BL/6 Ly5.1 mice are transduced at different MOIs with the gene trap or a control vector (SFFV-eGFP) and transplanted into lethally irradiated C57BL/6 Ly5.2 mice. Following engraftment (about 6 weeks after transplantation), mice are observed for a total of 6 months. Observation will involve two visual inspections per week, body weight control once every two weeks and a monthly analysis of peripheral blood including evaluation of engraftment, gp91 expression by flow cytometry and integration site analysis. After 8 months mice are sacrificed and subjected to a comprehensive post-mortem examination. Lung, spleen, thymus, lymph nodes, liver, heart, brain, kidney, bone marrow and peripheral blood are examined for tumors and/or leukemia. Genomic DNA is extracted from bone marrow and peripheral blood as well as from any enlarged organ. Gene trap insertions sites are identified and mapped to the genome. Common insertions sites in tumors or leukemias are identified by high throughput splinkerette and shot gun cloning. In a more sensitive assay, bone marrow from primary recipients is retransplanted into lethally irradiated secondary recipients, which are observed and analyzed as described above for a period of 3 months. The genotoxic potential of a particular vector is assessed by the loss of polyclonality and the emergence of oligo- or monoclonal cell populations.

The present invention provides proof of concept for a new gene therapy approach for monogenic disorders of hematopoietic cells.

The invention is further explained in the following Examples, which are, however not to be construed to limit the invention.

### Examples

Example 1: Gene Therapy of Chronic Granulomatous Disease (X-CGD). Two young adult X-CGD patients were treated in 2004 with gene modified cells in combination with non-myeloablative conditioning. Successful engraftment of gene transduced cells resulted in the eradication of pre-existing life-threatening bacterial and fungal infections. However, insertional activation of growth promoting genes triggered an increase in gene transduced cells in the peripheral blood of both patients Ott MG et al., Nature medicine 12, 401-409 (2006); Nature medicine 16, 198-204 (2010*).* One of these patients died 27 months after treatment due myelodysplasia in combination with multi-organ dysfunction. Although gene marking was still high at this time point, expression of the therapeutic gene, gp91phox, was minimal. The absence of transgene expression was due to CpG methylation within the viral LTR. Similar effects were also observed in the second patient. In both patients CpG methylation was restricted to the promoter region of the viral LTR, while CpG dinucleotides within the enhancer region of the viral LTRs were not methylated. As a consequence, gp91phox gene expression was suppressed but the capacity of the viral LTRs to transactivate nearby sequences was still intact. Indeed, we were able to detect cellular transcripts at predominant retroviral integration sites leading to clonal imbalance of gene marked cells in peripheral blood and bone marrow. Despite the severe adverse effects observed in our trial, the initial eradication of preexisting life-threatening infections and other clinical benefits experienced by both patients for more than two years after reinfusion of their autologous gene modified cells are impressive evidence that gene therapy can efficiently provide long-term cure and encourages the further development of gene therapy for the long-term correction of CGD. Advances in vector design with reduced genotoxicity and enhanced efficacy are thus highly desirable for a safe and effective application of gene therapy to CGD.

Example 2: Construction of gene trap vectors for the expression of gp91phox from cellular promoters. Figure 1 shows a typical reverse orientation splice acceptor (ROSA) gene trap vector consisting of a beta galactosidase/neomycinphosphor-transferase (βgeo) fusion gene flanked by an adenoviral splice acceptor (SA) and a bovine growth hormone polyadenylation sequence (pA) inserted in reverse transcriptional orientation between the LTRs of a 3'U3 promoter and enhancer deleted (SIN) Moloney murine leukemia virus. This basic gene trap design has been tested extensively in the past and is presently used for the high-throughput knock-out of genes expressed in ES cells. Insertion into a 5'intron simultaneously mutates and reports the expression of the trapped gene at the insertion site, and provides a DNA tag for the rapid identification of the disrupted gene. Presently, over 10,000 unique genes have been knocked out in murine ES cells using a variety of ROSA based gene trap vectors (www.genetrap.org) Nord AS et al., Nucleic acids research 34, D642-648 (2006). Database analysis indicated that the vast majority of the retroviral gene trap insertions were in the first and second introns of annotated genes (Figure 2), clearly reflecting the preference of retroviruses to insert into the 5'ends of genes.

Based on this classic gene trap configuration, four gene trap vectors were initially developed for in vitro testing: GT-IRESgp91 (SEQ ID NO:3), GT-SAgp91(SEQ ID NO:1), GT-EDN-SAgp91(SEQ ID NO:4 and GT-T2Agp91 (SEQ ID NO:2) shown in Figure 3. The GT-IRESgp91 vector contains an internal ribosomal entry site between the SA and gp91 to enable reading frame independent internal initiation of translation at the gp91 AUG. GT-SAgp91 has stop codons in all 3 reading frames upstream of gp91 to ensure translation initiation at the gp91 AUG and to prevent the fusion of upstream amino acids to gp91 that could interfere with its enzymatic function. As with GT-IRESgp91, the activation of GT-SAgp91 from insertions into expressed genes is reading frame independent. However, unlike GT-IRESgp91 which should be inducible from any insertion site within a gene, gp91 expression from GT-SAgp91 insertions is productive only from insertions downstream of non-coding 5'exons or 5'coding exons with short reading frames. The GT-EDN-SAgp91 vector contains an EDN enhancer element upstream of the SAgp91 cassette. In the GT-T2Agp91 vector the stop codons were replaced by a T2A polyprotein cleavage sequence sequence fused in frame to gp91 (Figure 3). In frame insertions of GT-T2Agp91 into any intron of an expressed gene induces a fusion transcript encoding a truncated and nonfunctional version of the cellular protein plus gp91, or - if the insertion is upstream of initiating methionines - just encodes the gp91. T2A mediates cotranslational cleavage of the truncated cellular protein from gp91 and thus ensures expression of native gp91.

In previous experiments, we found that the activity of reporter/selectable marker fusion proteins such as βgeo or hygromycin/EGFP could be significantly enhanced by T2A or P2A mediated cleavage (data not shown). Finally, all gp91 gene trap vectors contain woodchuck hepatitis virus posttranscriptional regulatory element (WPRE) posttranscriptional regulatory element upstream of the 3'LTR which has been shown to improve transgene expression in recipient cells Zufferey R et al., Journal of virology 73, 2886-2892 (1999); Schambach A et al., Gene therapy 13, 641-645 (2006).

Example 3: Transduction of X-CGD cells with gp91phox gene trap vectors. To test the gp91 transduction efficiency by the various gene trap vectors, we used the X-CGD human cell line PLB985 (X-CGD PLB985) derived from the myelomonocytic cell line PLB985 by knocking out Nox2. Like the parental PLB985 cells, X-CGD cells differentiate into mature neutrophils in the presence of dimethylformamide (DMF) or retinoic acid. Unlike the parental cells however, X-CGD neutrophils have no detectable NADPH oxidase activity and were therefore ideally suited for a first pass screen of gp91 transducing gene trap vectors.

GT-SAgp91, GT-EDN-SAgp91, GT-IRESgp91 and GT-T2Agp91 gibbon ape leukemia virus (GALV) pseudotyped retroviruses were produced by transient transfection in 293T cells. X-CGD cells were incubated with viral supernatants for 2 consecutive 24 hour intervals as previously described Becker S et al., Human gene therapy 9, 1561-1570 (1998). The SERS11.fes.91s.W gp91transducing gene therapy vector was used as a positive control Moreno-Carranza B et al., Gene therapy 16, 111-118 (2009). Transduction of gp91 was quantified by FACS using the monoclonal 7D5 anti-human gp91 antibody as previously described Becker S et al., Human gene therapy 9, 1561-1570 (1998). In two separate experiments the gp91 transduction efficiency with the GT-SAgp91 and GT-EDN-SAgp91 gene trap vectors was about 20 and 60 percent of that achieved with the control vector, respectively (Figure 4, left panel). Previous studies in animal models/clinical trials have shown that as little as 10 - 20% of functionally corrected neutrophils already protect X-CGD patients from infections Dinauer MC et al., Blood 97, 3738-3745 (2001).

GT-IRESgp91 and GT-T2Agp91 transduced only about 8 to 10% of the X-CGD cells (data not shown), presumably reflecting the well known restraint of internal ribosomal entry sites on downstream gene expression and the requirement of in frame insertions for productive gene trap events, respectively. We have not yet tested whether combining the three GT-T2Agp91 reading frame specific vectors would improve transduction.

To test whether neutrophils from GT-SAgp91 and GT-EDN-SAgp91 transduced X-CGD cells continue to express gp91, we exposed the cells to dimethylfoxide (DMSO) for 6 days. Figure 4 (right panel) shows that the neutrophils derived from both gene trap transduced cell populations continued to express gp91, indicating that gp91 expression is not lost upon differentiation. In line with this, nearly 100% of the neutrophils induced from FACS sorted gp91 positive X-CGD cells expressed gp91 (Figure 5A). Overall the results suggest that suggesting that the productive GT-SAgp91 insertions preferentially occurred in genes expressed in both cell populations. This interpretation is consistent with an Affymetrix chip analysis of 6,951 genes expressed in HL60 cells (from which the PLB985 cells were derived). Upon induction of differentiation by 12-O-tetradecanoylphorbol-13-acetate (TPA), 28% of the genes expressed in undifferentiated cells were still expressed in the differentiated cells, whereas only 9 and 7% were up- or down-regulated in differentiated cells, respectively (http://www.broad.mit.edu/cancer/software/ genecluster1/gc data np.zip).

Next, the dihydrorhodamine 123 (DHR) reduction assay was used to test whether the gp91 transduced and diffferentiated cells were still capable of producing superoxide after stimulation with phorbol-myristate-acetate (PMA). As shown in Figure 5B (right panel), PMA induced strong superoxide production in all transduced cells to levels as high as wild type levels, indicating that gp91 expressed from gene trap insertion sites fully retains its function.

Example 4: Characterization of gene trap events by 5'RACE and shot-gun cloning. To test whether GT-SAgp91vectors induced *bona fide* gene trap events, total RNA was extracted from gp91 positive cells and subjected to 5'RACE using primers complementary to gp91 (Figure 6A). The RACE products were cloned into pGMTeasy and transformed into E.coli DH5α. Forty Amp^{R} colonies were picked, expanded, mini-prepped and sequenced (Figure 6B). As expected from gene trapping in ES cells, most GT-SAgp91 insertions were in first or second introns of annotated genes and correctly spliced to the upstream exon(s). Upstream exons were either non-coding or encoded the first 1-21 amino acids of the trapped protein (Table 1). Overall, the results demonstrate that gp91 in GT-SAgp91 transduced X-CGD cells is expressed from the endogenous promoter/enhancer elements of trapped genes.

**Table 1: Unique genes trapped in GT-SAgp91 X-CGD cells**

| Clone # | Chromosome | ENSEMBL ID | Gene symbol | Intron | cd/nc* | Correct splicing |
|---|---|---|---|---|---|---|
| 1 | 13 | ENSG00000122026 | RL21 | 1 | Nc | yes |
| 3 | 1 | ENSG00000069424 | KCNAB2 | 1 | Nc | yes |
| 4 | 1 | ENSESTG00000031518 | PIK3CD | 2 | Nc | yes |
| 5 | 4 | ENSG00000109475 | RL34 | 1 | nc | yes |
| 12 | 1 | ENSG00000116584 | AGHREF 2 | 1 | c (21)** | yes |
| 15 | 11 | ENSG00000166439 | RNF169 | 1 | c(4) | ? |
| 20 | 2 | ENSG00000135624 | TCP1e | 1 | c(1) | yes |
| 23 | 11 | ENSG00000110367 | DDX6 | 1 | nc | yes |
| 27 | 15 | GENSCAN00000061406 | GABRB3 | *** | nc | yes |
| 32 | 5 | ENSG00000181163 | NPM1 | 2 | c (19) | yes |
| 33 | 1 | ENSG00000143669 | LYST | 1 | nc | yes |
| 35 | 22 | ENSG00000100345 | MYH9 | 1 | nc | yes |
| 38 | 8 | ENSG00000156467 | UQCRB | 1 | c (6) | yes |
| 39 | 11 | ENSG00000172725 | PTCA | 1 | c(1) | yes |
| W2-1 | 18 | ENSG00000134265 | Napg | 1 | c(18) | yes |
| W-8 | 6 | ENSG00000198755 | Rpl10A | 1 | c(1) | yes |
| E2-2 | 22 | ENSG00000100364 | C22orf9f | 1 | c (21) | yes |
| E5-1 | 4 | ENSG00000206823 | BC0179 93 | 1 | nc | no |
| E5-9 | 19 | ENSG00000172081 | Mobk12a | 1 | nc | yes |
| E5-11 | 21 | ENSG00000160201 | U2AF1 | 1 | c (14) | yes |

| | | | | | | |
|---|---|---|---|---|---|---|
| *coding/noncoding; ** number of aminoacids encoded by the upstream exon; *** Anti-sense transcript | | | | | | |

| Sequence Listing, Free Text | | |
|---|---|---|
| SEQ ID: | description[position/feature] | |
| 1 | vector GT-SAgp91 | |
| | 660 - 1064 | beta-lactamase (bla) |
| | 1162 - 1844 | ColE1 origin of replication |
| | 2255 - 2447 | 3' self incativating long terminal repeat (SIN-LTR) |
| | 2518 - 3030 | Woodchuck hepatitis virus posttranscriptional regulatory element (WPR) |
| | 3056 - 3179 | Adenoviral Splice Acceptor (SA) |
| | 3180 - 3190 | 3 x Stop Element |
| | 3191 - 3203 | Kozak Sequence |
| | 3200-4912 | GP91phox-S |
| | 4935 - 5214 | bovine Growth Hormone polyadenylation site (bGH pA) |
| | 5713 - 5857 | 5' long terminal repeat (LTR) |
| | 5858 - 6086 | Rous Sarcoma Virus (RSV) promoter |
| | 6095 - 6327 | Simian vacuolating virus 40 (SV40) enhancer |
| | | |
| 2 | vector GT-T2Agp91 | |
| | 660 - 1064 | beta-lactamase (bla) |
| | 1162 - 1844 | ColE1 origin of replication |
| | 2255 - 2447 | 3' self inactivating long terminal repeat (SIN-LTR) |
| | 2518 - 3030 | Woodchuck hepatitis virus posttranscriptional regulatory element (WPRE) |
| | 3114 - 3237 | Adenoviral Splice Acceptor (SA) |
| | 3251 - 3304 | Thosea asigna virus 2A-like (T2A) sequence |
| | 3305 - 5014 | GP91phox-S (without ATG) |
| | 5037 - 5316 | bovine Growth Hormone polyadenylation site (bHG pA) |
| | 5815 - 5959 | 5' long terminal repeat (LTR) |
| | 5960 - 6188 | Rous Sarcoma Virus (RSV) promoter |
| | 6197 - 6429 | Simian vacuolating virus 40 (SV40) enhancer |
| 3 | vector GT-EDN-SAgp91 | |
| | 660 - 1064 | beta-lactamase (bla) |
| | 1162 - 1844 | ColE1 origin of replication |
| | 2255 - 2447 | 3' self inactivating long terminal repeat (SIN-LTR) |
| | 2518 - 3030 | Woodchuck hepatitis virus posttranscriptional regulatory element (WPRE) |
| | 3114 - 3237 | Adenoviral Splice Acceptor (SA) |
| | 3262 - 3837 | Encephalomyocarditis virus internal ribosomal entry site (EMCV IRES) |
| | 3841 - 5553 | GP91phox-S |
| | 5576 - 5855 | bovine Growth Hormone polyadenylation site (bHG pA) |
| | 6354 - 6498 | 5' long terminal repeat (LTR) |
| | 6499 - 6727 | Rous Sarcoma Virus (RSV) promoter |
| | 6736 - 6968 | Simian vacuolating virus 40 (SV40) enhancer |
| | | |
| 4 | vector GT-EDN-SAgp91 | |
| | 660 - 1064 | beta-lactamase (bla) |
| | 1162 - 1844 | ColE1 origin of replication |
| | 2255 - 2447 | 3' self inactivating long terminal repeat (SIN-LTR) |
| | 2518 - 3030 | Woodchuck hepatitis virus posttranscriptional regulatory element (WPRE) |
| | 3103 - 3175 | Eosinophil-derived neurotoxin (EDN) enhancer element |
| | 3187 - 3310 | Adenoviral Splice Acceptor (SA) |
| | 3311 - 3321 | 3 x Stop Element |
| | 3322 - 3330 | Kozak Sequence |
| | 3331 - 5043 | GP91phox-S |
| | 5066 - 5345 | bovine Growth Hormone polyadenylation site (bGH pA) |
| | 5844 - 5988 | 5' long terminal repeat (LTR) |
| | 5989 - 6217 | Rous Sarcoma Virus (RSV) promoter |
| | 6226 - 6458 | Simian vacuolating virus 40 (SV40) enhancer |
| | | |
| 5 | vector GT-T2Agp91IRESSD | |
| | 660 - 1064 | beta-lactamase (bla) |
| | 1162 - 1844 | ColE1 origin of replication |
| | 2255 - 2447 | 3' self inactivating long terminal repeat (SIN-LTR) |
| | 2518 - 3030 | Woodchuck hepatitis virus posttranscriptional regulatory element (WPRE) |
| | 3114 - 3237 | Adenoviral Splice Acceptor (SA) |
| | 3251 - 3304 | Thosea asigna virus 2A-like (T2A) sequence |
| | 3305 - 5014 | GP91phox-S (without ATG) |
| | 5037 - 5612 | Encephalomyocarditis virus internal ribosomal entry site (EMCV IRES) |
| | 5613 - 5776 | Adenoviral Splice Donor (SD) |
| | 6275 - 6419 | 5' long terminal repeat (LTR) |
| | 6420 - 6648 | Rous Sarcoma Virus (RSV) promoter |
| | 6657 - 6889 | Simian vacuolating virus 40 (SV40) enhancer |

## Claims

1. A gene trap vector for use in gene therapy, which comprises a target gene cassette (TGC) and one or more enhancer elements, wherein said TGC comprises a promoterless target gene flanked by an upstream 3' splice acceptor (SA) site and a downstream transcriptional termination (polyA) sequence.

2. The gene trap vector for use in gene therapy of claim 1, wherein the target gene is a nucleic acid sequence for correcting a genetic defect of a patient, preferably
(i) is an intact gene for expression in a patient with a genetic loss of function mutation, preferably is a gene encoding coagulation factor VIII, coagulation factor IX, adenosine deaminase, common immunoglobulin gamma chain, adrenoleukodystrophy protein, hemoglobin etc., most preferably is encoding gp91phox encoded by the nucleic acid sequence of 3200-4912 of SEQ ID NO: 1, or
(ii) is a gene inactivating sequence for the knock-out of a gene expression in a patient, most preferably is an interfering short hairpin (shRNA), micro RNA (miRNA) or an antisense transcript.

3. The gene trap vector for use in gene therapy of claim 1 or 2, wherein one or more tissue specific enhancer elements are located upstream or downstream of the TGC.

4. The gene trap vector for use in gene therapy of any one of claims 1 to 3, wherein the target gene (i) is fused in frame to an upstream polyprotein cleavage sequence, preferably the cleavage sequence is P2A or T2A from Picorna virus; or (ii) is fused to an upstream internal ribosomal entry site (IRES)

5. The gene trap vector for use in gene therapy of any one of claims 1 to 5, wherein the polyA sequence of the TGC is replaced by a 5'splice donor site.

6. The gene trap vector for use in gene therapy of any one of claims 1 to 5, wherein the TGC and the one or more enhancer elements are contained in a genome integrating plasmid or virus, preferably in a self-inactivating lentivirus, most preferably in a self-inactivating retrovirus.

7. The gene trap vector for use in gene therapy of any one of claims 1 to 6, wherein the one or more enhancer elements
(i) contain at least one binding site for a transcription activating factor; and/or
(ii) contain binding sites that bind transcription activation factors in a sequence-specific manner, preferably the binding sites are arranged as tandem repeats; and/or
(iii) comprise tissue specific transcription factor binding elements, preferably the c-fms regulatory intronic element (FIRE) from the second intron of the CSF1-receptor encoding gene, , the CD68 enhancer from the first intron of the gene encoding microsialin, most preferably the Eosinophil-Derived Neurotoxin (EDN) enhancer from the first intron of the EDN encoding gene; and/or
(iv) are tandem repeats of AP-1, AP-2, CRE, SRE, NF-kB, SRF, SP1, Oct1, Oct2, Oct3, Oct4 transcription factor binding sites, preferably the enhancer element is an Oct-4 responsive enhancer element.

8. The gene trap vector for use in gene therapy of any one of claims 1 to 7, further comprising a selectable marker and/or a reporter gene, preferably the selectable marker is selected from the P140K-O(6)-methylguanine-methyltransferase (MGMT/P140K) gene, the puromycin resistance gene, the neomycinphosphotransferase fusion gene, the hygromycin resistance gene and the HSV thymidine kinase gene and the reporter gene is selected from a β-galactosidase gene, the enhanced greeen fluorescence protein (egfp) and the luciferase gene.

9. The gene trap vector for use in gene therapy of claim 8, wherein the selectable marker or reporter gene is inserted into the TGC upstream or downstream of the target gene using a 5' IRES or a polyprotein cleavage sequence.

10. The gene trap vector for use in gene therapy of claim 8, wherein the selectable marker is the MGMT/P140K gene.

11. The gene trap vector for use in gene therapy of any one of claims 1 to 10, which for the correction of chronic granulomatous disease and has the structure depicted in Fig. 3 or 7 and has a sequence shown in SEQ ID NOs: 1 to 5.

12. A gene trap vector, which comprises a target gene cassette (TGC) and one or more enhancer elements, wherein said TGC comprises a promoterless target gene, which is a nucleic acid sequence for correcting a genetic defect of a patient, flanked by an upstream 3' splice acceptor (SA) site and a downstream transcriptional termination (polyA) sequence.

13. The gene trap vector of claim 12, which is as defined in claims 2 to 11.

14. Use of a gene trap vector as defined in claims 1 to 13 for preparing a medicament for performing gene therapy.

15. A method for gene therapy comprising administering a patient in need of a gene therapy a gene trap vector as defined in claims 1 to 13.


SEQUENCE LISTING

<110>  von Melchner, Harald
       Grez, Manuel

<120>  Gene Trapping for Use in Gene Therapy

<130>  100452ep

<160>  5

<170>  PatentIn version 3.3

<210>  1
<211>  6837
<212>  DNA
<213>  Artificial

<220>
<223>  vector GT-SAgp91

<400>  1
cttgaagacg aaagggcctc gtgatacgcc tatttttata ggttaatgtc atgataataa      60

tggtttctta gacgtcaggt ggcacttttc ggggaaatgt gcgcggaacc cctatttgtt     120

tatttttcta aatacattca aatatgtatc cgctcatgag acaataaccc tgataaatgc     180

ttcaataata ttgaaaaagg aagagtatga gtattcaaca tttccgtgtc gcccttattc     240

ccttttttgc ggcattttgc cttcctgttt ttgctcaccc agaaacgctg gtgaaagtaa     300

aagatgctga agatcagttg ggtgcacgag tgggttacat cgaactggat ctcaacagcg     360

gtaagatcct tgagagtttt cgccccgaag aacgttttcc aatgatgagc acttttaaag     420

ttctgctatg tggcgcggta ttatcccgta ttgacgccgg gcaagagcaa ctcggtcgcc     480

gcatacacta ttctcagaat gacttggttg agtactcacc agtcacagaa aagcatctta     540

cggatggcat gacagtaaga gaattatgca gtgctgccat aaccatgagt gataacactg     600

cggccaactt acttctgaca acgatcggag gaccgaagga gctaaccgct tttttgcaca     660

acatggggga tcatgtaact cgccttgatc gttgggaacc ggagctgaat gaagccatac     720

caaacgacga gcgtgacacc acgatgccag cagcaatggc aacaacgttg cgcaaactat     780

taactggcga actacttact ctagcttccc ggcaacaatt aatagactgg atggaggcgg     840

ataaagttgc aggaccactt ctgcgctcgg cccttccggc tggctggttt attgctgata     900

aatctggagc cggtgagcgt gggtctcgcg gtatcattgc agcactgggg ccagatggta     960

agccctcccg tatcgtagtt atctacacga cggggagtca ggcaactatg gatgaacgaa    1020

atagacagat cgctgagata ggtgcctcac tgattaagca ttggtaactg tcagaccaag    1080

tttactcata tatactttag attgatttaa aacttcattt ttaatttaaa aggatctagg    1140

tgaagatcct ttttgataat ctcatgacca aaatccctta acgtgagttt tcgttccact 1200

gagcgtcaga ccccgtagaa aagatcaaag gatcttcttg agatcctttt tttctgcgcg 1260

taatctgctg cttgcaaaca aaaaaaccac cgctaccagc ggtggtttgt ttgccggatc 1320

aagagctacc aactcttttt ccgaaggtaa ctggcttcag cagagcgcag ataccaaata 1380

ctgtccttct agtgtagccg tagttaggcc accacttcaa gaactctgta gcaccgccta 1440

catacctcgc tctgctaatc ctgttaccag tggctgctgc cagtggcgat aagtcgtgtc 1500

ttaccgggtt ggactcaaga cgatagttac cggataaggc gcagcggtcg ggctgaacgg 1560

ggggttcgtg cacacagccc agcttggagc gaacgaccta caccgaactg agatacctac 1620

agcgtgagca ttgagaaagc gccacgcttc ccgaagggag aaaggcggac aggtatccgg 1680

taagcggcag ggtcggaaca ggagagcgca cgagggagct tccaggggga aacgcctggt 1740

atctttatag tcctgtcggg tttcgccacc tctgacttga gcgtcgattt ttgtgatgct 1800

cgtcaggggg gcggagccta tggaaaaacg ccagcaacgc ggccttttta cggttcctgg 1860

ccttttgctg gccttttgct cacatgttct ttcctgcgtt atcccctgat tctgtggata 1920

accgtattac cgcctttgag tgagctgata ccgctcgccg cagccgaacg accgagcgca 1980

gcgagtcagt gagcgaggaa gcggaagagc gcccaatacg caaaccgcct ctccccgcgc 2040

gttggccgat tcattaatgc agctggcacg acaggtttcc cgactggaaa gcgggcagtg 2100

agcgcaacgc aattaatgtg agttagctca ctcattaggc accccaggct ttacacttta 2160

tgcttccggc tcgtatgttg tgtggaattg tgagcggata acaatttcac acaggaaaca 2220

gctatgacca tgattacgcc aaagctctcg agaatgaaag acccccgagg tgggcagtca 2280

atcactcaga ggagaccctc ccaaggaaca gcgagaccac gagtcggatg caactgcaag 2340

agggtttatt gagaacacgg gtacccgggc gactcagtct gtcggaggac tggcgcgccg 2400

agtgaggggt tgtgaattcg atatcgctag cggtggggtc tttcattccc ccctttttct 2460

ggagactaaa taaaatcttt tattctatct atggctcgtg ttaagcttta tcgaattcga 2520

caacaccacg gaattatcag tgcccagcaa cctagcccct gtccagcagc gggcaaggca 2580

ggcggcgatg agttctgccg tggcgatcgg gagggggaaa gcgaaagtcc cagaaaggag 2640

ttgacaggtg gtggcaatgc cccagccagt gggggttgcg tcagcaaaca cagagcacac 2700

cacgccacgt tgacggacaa cgggccacaa ctcctctaaa agagacagca accaggattt 2760

atacaaggag gagaaaacga aagccgtacg ggaagcaata gctagataca gaggctataa 2820

agcagcatat ccacacagcg taaaaggagc aacatagtta agaatatcag tcaatctttc 2880

acaaattttg taatccagag gttgattaac aggaacagag cgtaaataac gggaaagttt 2940

cttaacatgt ttgtcttgtg gcaatacacc tgaactagta attacatatc cctaaaaatg 3000

taaatgattg ccccaccatt ttgttttatt aacatttaaa tgtataccca aatcaagaaa 3060

aacagaacaa atatgggaat aaatggcggt aagatgctct cgcgaccaga tctgtagggc 3120

gcagtagtcc agggtttcct tgatgatgtc atacttatcc tgtccctttt ttttccacag 3180

ctcgcggttt gaggacaaac tcttcgcggt ctttccagtg gggatcgacg gtatcgattg 3240

attgattgag ccgccaccat gggcaactgg gccgtgaacg agggcctgag catcttcgtg 3300

atcctggtgt ggctgggcct gaacgtgttc ctgttcgtgt ggtactaccg ggtgtacgac 3360

atccccccca agttcttcta cacccggaag ctgctgggca gcgccctggc cctggccaga 3420

gcccctgccg cctgcctgaa cttcaactgc atgctgatcc tgctgcccgt gtgccggaac 3480

ctgctgtcct tcctgcgggg cagcagcgcc tgctgcagca ccagagtgcg gcggcagctg 3540

gaccggaacc tgaccttcca caagatggtg gcctggatga tcgccctgca cagcgccatc 3600

cacaccatcg cccacctgtt caacgtggag tggtgcgtga acgcccgggt gaacaacagc 3660

gacccctaca gcgtggccct gagcgagctg ggcgaccggc agaacgagag ctacctgaac 3720

ttcgcccgga agcggatcaa gaaccccgag ggcggcctgt acctggccgt gaccctgctg 3780

gccggcatca ccggcgtggt gatcaccctg tgcctgatcc tgatcatcac cagcagcacc 3840

aagaccatcc ggcggagcta cttcgaggtg ttctggtaca cccaccacct gttcgtgatc 3900

tttttcatcg gcctggccat ccacggcgcc gagcggatcg tgaggggcca gaccgccgag 3960

agcctggccg tgcacaacat caccgtgtgc gagcagaaaa tcagcgagtg gggcaagatc 4020

aaagagtgcc ccatccccca gttcgccggc aaccccccca tgacctggaa gtggatcgtg 4080

ggccccatgt tcctgtacct gtgcgagcgg ctggtgcggt tctggcggag ccagcagaaa 4140

gtggtgatta ccaaggtggt gacccacccc ttcaagacca tcgagctgca gatgaagaaa 4200

aagggcttca agatggaagt gggccagtac atctttgtga agtgccccaa ggtgtccaag 4260

ctggaatggc accccttcac cctgaccagc gcccctgaag aggacttctt cagcatccac 4320

atcagaatcg tgggcgactg gaccgagggc ctgttcaatg cctgcggctg cgacaagcag 4380

gaattccagg acgcctggaa gctgcccaag atcgccgtgg acggcccctt tggcaccgcc 4440

agcgaggacg tgttcagcta cgaggtggtg atgctggtcg gagccggcat cggcgtgacc 4500

cccttcgcca gcatcctgaa gagcgtgtgg tacaagtact gcaacaacgc caccaacctg 4560

aagctgaaga agatctactt ctactggctg tgccgggaca cccacgcctt cgagtggttc 4620

gccgatctgc tgcagctgct ggaaagccag atgcaggaac ggaacaacgc cggcttcctg 4680

agctacaaca tctacctgac cggctgggac gagagccagg ccaaccactt cgccgtgcac 4740

cacgacgagg aaaaggacgt gatcaccggc ctgaagcaga aaaccctgta cggcaggccc 4800

aactgggaca acgagtttaa gaccatcgcc agccagcacc ccaacacccg gatcggcgtg 4860

tttctgtgcg gccctgaggc cctggccgag acactgagca agcagagcat cagcaacagc 4920

gagagcggcc ccaggggcgt gcacttcatc ttcaacaaag aaaacttctg atgagtatac 4980

atttgtatct agagctcgct gatcagcctc gactgtgcct tctagttgcc agccatctgt 5040

tgtttgcccc tcccccgtgc cttccttgac cctggaaggt gccactccca ctgtcctttc 5100

ctaataaaat gaggaaattg catcgcattg tctgagtagg tgtcattcta ttctgggggg 5160

tggggtgggg caggacagca agggggagga ttgggaagac aatagcaggc atgctgggga 5220

tgcggtgggc tctatggctt ctgagacgga aagaaccagc tggggctcga tcctctagag 5280

cggccgctat tttcagacaa tacagaacca cgtcagacag agacaacaca gaacgatgct 5340

gcagcagaca agacgcgcgg cgcggtttcg gcgtaaaacc gaaagcaaaa attcagacgg 5400

gggcgggaag tgtctctgtc tctcgtcccc caacaaagcc acgtacccct cggacagtgg 5460

cggagctcca aaaagtccga ctcgggtagg ttaactgata cagaatgggc cacaaaacgg 5520

gcccccgagg ccgctgggac gtctcccagg ggctgcggcc gggaatacga actcgtcagt 5580

tcttccgcgg gaccgccaga tacagatcta gttagccaac tagtacagac gcaggcgcaa 5640

acactagatg ccggcacaaa cacgcccaaa gacagagaca gacacgaaac gaccgctggc 5700

cagcttacct cccggcgggg gggtcggtgg tccctgggca ggggtctcca aatctcggtg 5760

gaacctccaa atgaaagacc cccgaggtgg gcagtcaatc aatctgagga gaccctccca 5820

aggatcagcg agtccacgat tcggatgcaa acagcaagag gctttattgg gaatacgggt 5880

acccgggcga cgcagtcaat cggaggactg gcgcgtttat tgtatcgagc taggcactta 5940

aatacaatat ctctgcaatg cggcaattca gtggttcgtc caatccatgt cagacccgtc 6000

tgttgccttc ctaataaggc acgatcgtac caccttactt ccaccaatcg gcatgcacgg 6060

tgctttttct ctccttgtaa ggcatgttgc taactcatcg ttaccatgtt gcaagactac 6120

aagagtattg cataagacta cattcttaag ggactatggt tgctgactaa ttgagatgca 6180

tgctttgcat acttctgcct gctggggagc ctggggactt tccacacctg gttgctgact 6240

aattgagatg catgctttgc atacttctgc ctgctgggga gcctggggac tttccacacc 6300

ctaactgaca cacattccac agctggttct ttccgcctca gaaggtacct aaccaagttc 6360

ctctttcaga ggttatttca ggccacgcgt cttaagctag agtactaatt cactggccgt 6420

cgttttacaa cgtcgtgact gggaaaaccc tggcgttacc caacttaatc gccttgcagc 6480

acatccccct ttcgccagct ggcgtaatag cgaagaggcc cgcaccgatc gcccttccca 6540

acagttgcgc agcctgaatg gcgaatggcg cctgatgcgg tattttctcc ttacgcatct 6600

gtgcggtatt tcacaccgca tatatggtgc actctcagta caatctgctc tgatgccgca 6660

tagtaactat cgctacgtga ctgggtcatg gctgcgcccc gacacccgcc aacacccgct 6720

gacgcgccct gacgggcttg tctgctcccg gcatccgctt acagacaagc tgtgaccgtc 6780

tccgggagct gcatgtgtca gaggttttca ccgtcatcac cgaaacgcgc gaggcag 6837

<210> 2
<211> 6880
<212> DNA
<213> Artificial

<220>
<223> vector GT-T2Agp91

<400> 2
cttgaagacg aaagggcctc gtgatacgcc tatttttata ggttaatgtc atgataataa 60

tggtttctta gacgtcaggt ggcacttttc ggggaaatgt gcgcggaacc cctatttgtt 120

tatttttcta aatacattca aatatgtatc cgctcatgag acaataaccc tgataaatgc 180

ttcaataata ttgaaaaagg aagagtatga gtattcaaca tttccgtgtc gcccttattc 240

ccttttttgc ggcattttgc cttcctgttt ttgctcaccc agaaacgctg gtgaaagtaa 300

aagatgctga agatcagttg ggtgcacgag tgggttacat cgaactggat ctcaacagcg 360

gtaagatcct tgagagtttt cgccccgaag aacgttttcc aatgatgagc acttttaaag 420

ttctgctatg tggcgcggta ttatcccgta ttgacgccgg gcaagagcaa ctcggtcgcc 480

gcatacacta ttctcagaat gacttggttg agtactcacc agtcacagaa aagcatctta 540

cggatggcat gacagtaaga gaattatgca gtgctgccat aaccatgagt gataacactg 600

cggccaactt acttctgaca acgatcggag gaccgaagga gctaaccgct tttttgcaca 660

acatggggga tcatgtaact cgccttgatc gttgggaacc ggagctgaat gaagccatac 720

caaacgacga gcgtgacacc acgatgccag cagcaatggc aacaacgttg cgcaaactat 780

taactggcga actacttact ctagcttccc ggcaacaatt aatagactgg atggaggcgg 840

ataaagttgc aggaccactt ctgcgctcgg cccttccggc tggctggttt attgctgata 900

aatctggagc cggtgagcgt gggtctcgcg gtatcattgc agcactgggg ccagatggta 960

agccctcccg tatcgtagtt atctacacga cggggagtca ggcaactatg gatgaacgaa 1020

atagacagat cgctgagata ggtgcctcac tgattaagca ttggtaactg tcagaccaag 1080

tttactcata tatactttag attgatttaa aacttcattt ttaatttaaa aggatctagg 1140

tgaagatcct ttttgataat ctcatgacca aaatccctta acgtgagttt tcgttccact 1200

gagcgtcaga ccccgtagaa aagatcaaag gatcttcttg agatcctttt tttctgcgcg 1260

taatctgctg cttgcaaaca aaaaaaccac cgctaccagc ggtggtttgt ttgccggatc 1320

aagagctacc aactcttttt ccgaaggtaa ctggcttcag cagagcgcag ataccaaata 1380

ctgtccttct agtgtagccg tagttaggcc accacttcaa gaactctgta gcaccgccta 1440

catacctcgc tctgctaatc ctgttaccag tggctgctgc cagtggcgat aagtcgtgtc 1500

ttaccgggtt ggactcaaga cgatagttac cggataaggc gcagcggtcg ggctgaacgg 1560

ggggttcgtg cacacagccc agcttggagc gaacgaccta caccgaactg agatacctac 1620

agcgtgagca ttgagaaagc gccacgcttc ccgaagggag aaaggcggac aggtatccgg 1680

taagcggcag ggtcggaaca ggagagcgca cgagggagct tccaggggga aacgcctggt 1740

atctttatag tcctgtcggg tttcgccacc tctgacttga gcgtcgattt ttgtgatgct 1800

cgtcaggggg gcggagccta tggaaaaacg ccagcaacgc ggccttttta cggttcctgg 1860

ccttttgctg gccttttgct cacatgttct ttcctgcgtt atcccctgat tctgtggata 1920

accgtattac cgcctttgag tgagctgata ccgctcgccg cagccgaacg accgagcgca 1980

gcgagtcagt gagcgaggaa gcggaagagc gcccaatacg caaaccgcct ctccccgcgc 2040

gttggccgat tcattaatgc agctggcacg acaggtttcc cgactggaaa gcgggcagtg 2100

agcgcaacgc aattaatgtg agttagctca ctcattaggc accccaggct ttacacttta 2160

tgcttccggc tcgtatgttg tgtggaattg tgagcggata acaatttcac acaggaaaca 2220

gctatgacca tgattacgcc aaagctctcg agaatgaaag acccccgagg tgggcagtca 2280

atcactcaga ggagaccctc ccaaggaaca gcgagaccac gagtcggatg caactgcaag 2340

agggtttatt gagaacacgg gtacccgggc gactcagtct gtcggaggac tggcgcgccg 2400

agtgaggggt tgtgaattcg atatcgctag cggtggggtc tttcattccc ccctttttct 2460

ggagactaaa taaaatcttt tattctatct atggctcgtg ttaagcttta tcgaattcga 2520

caacaccacg gaattatcag tgcccagcaa cctagcccct gtccagcagc gggcaaggca 2580

ggcggcgatg agttctgccg tggcgatcgg gagggggaaa gcgaaagtcc cagaaaggag 2640

ttgacaggtg gtggcaatgc cccagccagt gggggttgcg tcagcaaaca cagagcacac 2700

cacgccacgt tgacggacaa cgggccacaa ctcctctaaa agagacagca accaggattt 2760

atacaaggag gagaaaacga aagccgtacg ggaagcaata gctagataca gaggctataa 2820

agcagcatat ccacacagcg taaaaggagc aacatagtta agaatatcag tcaatctttc 2880

acaaattttg taatccagag gttgattaac aggaacagag cgtaaataac gggaaagttt 2940

cttaacatgt ttgtcttgtg gcaatacacc tgaactagta attacatatc cctaaaaatg 3000

taaatgattg ccccaccatt ttgttttatt aacatttaaa tgtataccca aatcaagaaa 3060

aacagaacaa atatgggaat aaatggcggt aagatgctct cgcgaccaga tctgtagggc 3120

gcagtagtcc agggtttcct tgatgatgtc atacttatcc tgtccctttt ttttccacag 3180

ctcgcggttg aggacaaact cttcgcggtc tttccagtgg ggatcgacgg tatcgataag 3240

cttgatctcc gagggcagag gaagtcttct aacatgcggt gacgtggagg agaatcccgg 3300

ccctggcaac tgggccgtga acgagggcct gagcatcttc gtgatcctgg tgtggctggg 3360

cctgaacgtg ttcctgttcg tgtggtacta ccgggtgtac gacatccccc ccaagttctt 3420

ctacacccgg aagctgctgg gcagcgccct ggccctggcc agagcccctg ccgcctgcct 3480

gaacttcaac tgcatgctga tcctgctgcc cgtgtgccgg aacctgctgt ccttcctgcg 3540

gggcagcagc gcctgctgca gcaccagagt gcggcggcag ctggaccgga acctgacctt 3600

ccacaagatg gtggcctgga tgatcgccct gcacagcgcc atccacacca tcgcccacct 3660

gttcaacgtg gagtggtgcg tgaacgcccg ggtgaacaac agcgacccct acagcgtggc 3720

cctgagcgag ctgggcgacc ggcagaacga gagctacctg aacttcgccc ggaagcggat 3780

caagaacccc gagggcggcc tgtacctggc cgtgaccctg ctggccggca tcaccggcgt 3840

ggtgatcacc ctgtgcctga tcctgatcat caccagcagc accaagacca tccggcggag 3900

ctacttcgag gtgttctggt acacccacca cctgttcgtg atctttttca tcggcctggc 3960

catccacggc gccgagcgga tcgtgagggg ccagaccgcc gagagcctgg ccgtgcacaa 4020

catcaccgtg tgcgagcaga aaatcagcga gtggggcaag atcaaagagt gccccatccc 4080

ccagttcgcc ggcaaccccc ccatgacctg gaagtggatc gtgggcccca tgttcctgta 4140

cctgtgcgag cggctggtgc ggttctggcg gagccagcag aaagtggtga ttaccaaggt 4200

ggtgacccac cccttcaaga ccatcgagct gcagatgaag aaaaagggct tcaagatgga 4260

agtgggccag tacatctttg tgaagtgccc caaggtgtcc aagctggaat ggcacccctt 4320

caccctgacc agcgcccctg aagaggactt cttcagcatc cacatcagaa tcgtgggcga 4380

ctggaccgag ggcctgttca atgcctgcgg ctgcgacaag caggaattcc aggacgcctg 4440

gaagctgccc aagatcgccg tggacggccc ctttggcacc gccagcgagg acgtgttcag 4500

ctacgaggtg gtgatgctgg tcggagccgg catcggcgtg acccccttcg ccagcatcct 4560

gaagagcgtg tggtacaagt actgcaacaa cgccaccaac ctgaagctga agaagatcta 4620

cttctactgg ctgtgccggg acacccacgc cttcgagtgg ttcgccgatc tgctgcagct 4680

gctggaaagc cagatgcagg aacggaacaa cgccggcttc ctgagctaca acatctacct 4740

gaccggctgg gacgagagcc aggccaacca cttcgccgtg caccacgacg aggaaaagga 4800

cgtgatcacc ggcctgaagc agaaaaccct gtacggcagg cccaactggg acaacgagtt 4860

taagaccatc gccagccagc accccaacac ccggatcggc gtgtttctgt gcggccctga 4920

ggccctggcc gagacactga gcaagcagag catcagcaac agcgagagcg gccccagggg 4980

cgtgcacttc atcttcaaca aagaaaactt ctgatgagta tacatttgta tctagagctc 5040

gctgatcagc ctcgactgtg ccttctagtt gccagccatc tgttgtttgc ccctcccccg 5100

tgccttcctt gaccctggaa ggtgccactc ccactgtcct ttcctaataa aatgaggaaa 5160

ttgcatcgca ttgtctgagt aggtgtcatt ctattctggg gggtggggtg gggcaggaca 5220

gcaaggggga ggattgggaa gacaatagca ggcatgctgg ggatgcggtg ggctctatgg 5280

cttctgagac ggaaagaacc agctggggct cgatcctcta gagcggccgc tattttcaga 5340

caatacagaa ccacgtcaga cagagacaac acagaacgat gctgcagcag acaagacgcg 5400

cggcgcggtt tcggcgtaaa accgaaagca aaaattcaga cgggggcggg aagtgtctct 5460

gtctctcgtc ccccaacaaa gccacgtacc cctcggacag tggcggagct ccaaaaagtc 5520

cgactcgggt aggttaactg atacagaatg ggccacaaaa cgggcccccg aggccgctgg 5580

gacgtctccc aggggctgcg gccgggaata cgaactcgtc agttcttccg cgggaccgcc 5640

agatacagat ctagttagcc aactagtaca gacgcaggcg caaacactag atgccggcac 5700

aaacacgccc aaagacagag acagacacga aacgaccgct ggccagctta cctcccggcg 5760

ggggggtcgg tggtccctgg gcaggggtct ccaaatctcg gtggaacctc caaatgaaag 5820

acccccgagg tgggcagtca atcaatctga ggagaccctc ccaaggatca gcgagtccac 5880

gattcggatg caaacagcaa gaggctttat tgggaatacg ggtacccggg cgacgcagtc 5940

aatcggagga ctggcgcgtt tattgtatcg agctaggcac ttaaatacaa tatctctgca 6000

atgcggcaat tcagtggttc gtccaatcca tgtcagaccc gtctgttgcc ttcctaataa 6060

ggcacgatcg taccacctta cttccaccaa tcggcatgca cggtgctttt tctctccttg 6120

taaggcatgt tgctaactca tcgttaccat gttgcaagac tacaagagta ttgcataaga 6180

ctacattctt aagggactat ggttgctgac taattgagat gcatgctttg catacttctg 6240

cctgctgggg agcctgggga ctttccacac ctggttgctg actaattgag atgcatgctt 6300

tgcatacttc tgcctgctgg ggagcctggg gactttccac accctaactg acacacattc 6360

cacagctggt tctttccgcc tcagaaggta cctaaccaag ttcctctttc agaggttatt 6420

tcaggccacg cgtcttaagc tagagtacta attcactggc cgtcgtttta caacgtcgtg 6480

actgggaaaa ccctggcgtt acccaactta atcgccttgc agcacatccc cctttcgcca 6540

gctggcgtaa tagcgaagag gcccgcaccg atcgcccttc ccaacagttg cgcagcctga 6600

atggcgaatg gcgcctgatg cggtattttc tccttacgca tctgtgcggt atttcacacc 6660

gcatatatgg tgcactctca gtacaatctg ctctgatgcc gcatagtaac tatcgctacg 6720

tgactgggtc atggctgcgc cccgacaccc gccaacaccc gctgacgcgc cctgacgggc 6780

ttgtctgctc ccggcatccg cttacagaca agctgtgacc gtctccggga gctgcatgtg 6840

tcagaggttt tcaccgtcat caccgaaacg cgcgaggcag 6880

<210> 3
<211> 7419
<212> DNA
<213> Artificial

<220>
<223> vector GT-IRESgp91

<400> 3
cttgaagacg aaagggcctc gtgatacgcc tatttttata ggttaatgtc atgataataa 60

tggtttctta gacgtcaggt ggcacttttc ggggaaatgt gcgcggaacc cctatttgtt 120

tatttttcta aatacattca aatatgtatc cgctcatgag acaataaccc tgataaatgc 180

ttcaataata ttgaaaaagg aagagtatga gtattcaaca tttccgtgtc gcccttattc 240

ccttttttgc ggcattttgc cttcctgttt ttgctcaccc agaaacgctg gtgaaagtaa 300

aagatgctga agatcagttg ggtgcacgag tgggttacat cgaactggat ctcaacagcg 360

gtaagatcct tgagagtttt cgccccgaag aacgttttcc aatgatgagc acttttaaag 420

ttctgctatg tggcgcggta ttatcccgta ttgacgccgg gcaagagcaa ctcggtcgcc 480

gcatacacta ttctcagaat gacttggttg agtactcacc agtcacagaa aagcatctta 540

cggatggcat gacagtaaga gaattatgca gtgctgccat aaccatgagt gataacactg 600

cggccaactt acttctgaca acgatcggag gaccgaagga gctaaccgct tttttgcaca 660

acatggggga tcatgtaact cgccttgatc gttgggaacc ggagctgaat gaagccatac 720

caaacgacga gcgtgacacc acgatgccag cagcaatggc aacaacgttg cgcaaactat 780

taactggcga actacttact ctagcttccc ggcaacaatt aatagactgg atggaggcgg 840

ataaagttgc aggaccactt ctgcgctcgg cccttccggc tggctggttt attgctgata 900

aatctggagc cggtgagcgt gggtctcgcg gtatcattgc agcactgggg ccagatggta 960

agccctcccg tatcgtagtt atctacacga cggggagtca ggcaactatg gatgaacgaa 1020

atagacagat cgctgagata ggtgcctcac tgattaagca ttggtaactg tcagaccaag 1080

tttactcata tatactttag attgatttaa aacttcattt ttaatttaaa aggatctagg 1140

tgaagatcct ttttgataat ctcatgacca aaatccctta acgtgagttt tcgttccact 1200

gagcgtcaga ccccgtagaa aagatcaaag gatcttcttg agatcctttt tttctgcgcg 1260

taatctgctg cttgcaaaca aaaaaaccac cgctaccagc ggtggtttgt ttgccggatc 1320

aagagctacc aactcttttt ccgaaggtaa ctggcttcag cagagcgcag ataccaaata 1380

ctgtccttct agtgtagccg tagttaggcc accacttcaa gaactctgta gcaccgccta 1440

catacctcgc tctgctaatc ctgttaccag tggctgctgc cagtggcgat aagtcgtgtc 1500

ttaccgggtt ggactcaaga cgatagttac cggataaggc gcagcggtcg ggctgaacgg 1560

ggggttcgtg cacacagccc agcttggagc gaacgaccta caccgaactg agatacctac 1620

agcgtgagca ttgagaaagc gccacgcttc ccgaagggag aaaggcggac aggtatccgg 1680

taagcggcag ggtcggaaca ggagagcgca cgagggagct tccaggggga aacgcctggt 1740

atctttatag tcctgtcggg tttcgccacc tctgacttga gcgtcgattt ttgtgatgct 1800

cgtcaggggg gcggagccta tggaaaaacg ccagcaacgc ggccttttta cggttcctgg 1860

ccttttgctg gccttttgct cacatgttct ttcctgcgtt atcccctgat tctgtggata 1920

accgtattac cgcctttgag tgagctgata ccgctcgccg cagccgaacg accgagcgca 1980

gcgagtcagt gagcgaggaa gcggaagagc gcccaatacg caaaccgcct ctccccgcgc 2040

gttggccgat tcattaatgc agctggcacg acaggtttcc cgactggaaa gcgggcagtg 2100

agcgcaacgc aattaatgtg agttagctca ctcattaggc accccaggct ttacacttta 2160

tgcttccggc tcgtatgttg tgtggaattg tgagcggata acaatttcac acaggaaaca 2220

gctatgacca tgattacgcc aaagctctcg agaatgaaag acccccgagg tgggcagtca 2280

atcactcaga ggagaccctc ccaaggaaca gcgagaccac gagtcggatg caactgcaag 2340

agggtttatt gagaacacgg gtacccgggc gactcagtct gtcggaggac tggcgcgccg 2400

agtgaggggt tgtgaattcg atatcgctag cggtggggtc tttcattccc ccctttttct 2460

ggagactaaa taaaatcttt tattctatct atggctcgtg ttaagcttta tcgaattcga 2520

caacaccacg gaattatcag tgcccagcaa cctagcccct gtccagcagc gggcaaggca 2580

ggcggcgatg agttctgccg tggcgatcgg gagggggaaa gcgaaagtcc cagaaaggag 2640

ttgacaggtg gtggcaatgc cccagccagt gggggttgcg tcagcaaaca cagagcacac 2700

cacgccacgt tgacggacaa cgggccacaa ctcctctaaa agagacagca accaggattt 2760

atacaaggag gagaaaacga aagccgtacg ggaagcaata gctagataca gaggctataa 2820

agcagcatat ccacacagcg taaaaggagc aacatagtta agaatatcag tcaatctttc 2880

acaaattttg taatccagag gttgattaac aggaacagag cgtaaataac gggaaagttt 2940

cttaacatgt ttgtcttgtg gcaatacacc tgaactagta attacatatc cctaaaaatg 3000

taaatgattg ccccaccatt ttgttttatt aacatttaaa tgtataccca aatcaagaaa 3060

aacagaacaa atatgggaat aaatggcggt aagatgctct cgcgaccaga tctgtagggc 3120

gcagtagtcc agggtttcct tgatgatgtc atacttatcc tgtccctttt ttttccacag 3180

ctcgcggttg aggacaaact cttcgcggtc tttccagtgg ggatcgacgg tatcgattga 3240

ttgattgagc cgccaccatg gcgcccctct ccctcccccc cccctaacgt tactggccga 3300

agccgcttgg aataaggccg gtgtgcgttt gtctatatgt tattttccac catattgccg 3360

tcttttggca atgtgagggc ccggaaacct ggccctgtct tcttgacgag cattcctagg 3420

ggtctttccc ctctcgccaa aggaatgcaa ggtctgttga atgtcgtgaa ggaagcagtt 3480

cctctggaag cttcttgaag acaaacaacg tctgtagcga ccctttgcag gcagcggaac 3540

cccccacctg gcgacaggtg cctctgcggc caaaagccac gtgtataaga tacacctgca 3600

aaggcggcac aaccccagtg ccacgttgtg agttggatag ttgtggaaag agtcaaatgg 3660

ctctcctcaa gcgtattcaa caaggggctg aaggatgccc agaaggtacc ccattgtatg 3720

ggatctgatc tggggcctcg gtgcacatgc tttacatgtg tttagtcgag gttaaaaaac 3780

gtctaggccc cccgaaccac ggggacgtgg ttttcctttg aaaaacacga tgataatacc 3840

atgggcaact gggccgtgaa cgagggcctg agcatcttcg tgatcctggt gtggctgggc 3900

ctgaacgtgt tcctgttcgt gtggtactac cgggtgtacg acatcccccc caagttcttc 3960

tacacccgga agctgctggg cagcgccctg gccctggcca gagcccctgc cgcctgcctg 4020

aacttcaact gcatgctgat cctgctgccc gtgtgccgga acctgctgtc cttcctgcgg 4080

ggcagcagcg cctgctgcag caccagagtg cggcggcagc tggaccggaa cctgaccttc 4140

cacaagatgg tggcctggat gatcgccctg cacagcgcca tccacaccat cgcccacctg 4200

ttcaacgtgg agtggtgcgt gaacgcccgg gtgaacaaca gcgaccccta cagcgtggcc 4260

ctgagcgagc tgggcgaccg gcagaacgag agctacctga acttcgcccg gaagcggatc 4320

aagaaccccg agggcggcct gtacctggcc gtgaccctgc tggccggcat caccggcgtg 4380

gtgatcaccc tgtgcctgat cctgatcatc accagcagca ccaagaccat ccggcggagc 4440

tacttcgagg tgttctggta cacccaccac ctgttcgtga tctttttcat cggcctggcc 4500

atccacggcg ccgagcggat cgtgaggggc cagaccgccg agagcctggc cgtgcacaac 4560

atcaccgtgt gcgagcagaa aatcagcgag tggggcaaga tcaaagagtg ccccatcccc 4620

cagttcgccg gcaacccccc catgacctgg aagtggatcg tgggccccat gttcctgtac 4680

ctgtgcgagc ggctggtgcg gttctggcgg agccagcaga aagtggtgat taccaaggtg 4740

gtgacccacc ccttcaagac catcgagctg cagatgaaga aaaagggctt caagatggaa 4800

gtgggccagt acatctttgt gaagtgcccc aaggtgtcca agctggaatg gcaccccttc 4860

accctgacca gcgcccctga agaggacttc ttcagcatcc acatcagaat cgtgggcgac 4920

tggaccgagg gcctgttcaa tgcctgcggc tgcgacaagc aggaattcca ggacgcctgg 4980

aagctgccca agatcgccgt ggacggcccc tttggcaccg ccagcgagga cgtgttcagc 5040

tacgaggtgg tgatgctggt cggagccggc atcggcgtga cccccttcgc cagcatcctg 5100

aagagcgtgt ggtacaagta ctgcaacaac gccaccaacc tgaagctgaa gaagatctac 5160

ttctactggc tgtgccggga cacccacgcc ttcgagtggt tcgccgatct gctgcagctg 5220

ctggaaagcc agatgcagga acggaacaac gccggcttcc tgagctacaa catctacctg 5280

accggctggg acgagagcca ggccaaccac ttcgccgtgc accacgacga ggaaaaggac 5340

gtgatcaccg gcctgaagca gaaaaccctg tacggcaggc ccaactggga caacgagttt 5400

aagaccatcg ccagccagca ccccaacacc cggatcggcg tgtttctgtg cggccctgag 5460

gccctggccg agacactgag caagcagagc atcagcaaca gcgagagcgg ccccaggggc 5520

gtgcacttca tcttcaacaa agaaaacttc tgatgagtat acatttgtat ctagagctcg 5580

ctgatcagcc tcgactgtgc cttctagttg ccagccatct gttgtttgcc cctcccccgt 5640

gccttccttg accctggaag gtgccactcc cactgtcctt tcctaataaa atgaggaaat 5700

tgcatcgcat tgtctgagta ggtgtcattc tattctgggg ggtggggtgg ggcaggacag 5760

caagggggag gattgggaag acaatagcag gcatgctggg gatgcggtgg gctctatggc 5820

ttctgagacg gaaagaacca gctggggctc gatcctctag agcggccgct attttcagac 5880

aatacagaac cacgtcagac agagacaaca cagaacgatg ctgcagcaga caagacgcgc 5940

ggcgcggttt cggcgtaaaa ccgaaagcaa aaattcagac gggggcggga agtgtctctg 6000

tctctcgtcc cccaacaaag ccacgtaccc ctcggacagt ggcggagctc caaaaagtcc 6060

gactcgggta ggttaactga tacagaatgg gccacaaaac gggcccccga ggccgctggg 6120

acgtctccca ggggctgcgg ccgggaatac gaactcgtca gttcttccgc gggaccgcca 6180

gatacagatc tagttagcca actagtacag acgcaggcgc aaacactaga tgccggcaca 6240

aacacgccca aagacagaga cagacacgaa acgaccgctg gccagcttac ctcccggcgg 6300

gggggtcggt ggtccctggg caggggtctc caaatctcgg tggaacctcc aaatgaaaga 6360

cccccgaggt gggcagtcaa tcaatctgag gagaccctcc caaggatcag cgagtccacg 6420

attcggatgc aaacagcaag aggctttatt gggaatacgg gtacccgggc gacgcagtca 6480

atcggaggac tggcgcgttt attgtatcga gctaggcact taaatacaat atctctgcaa 6540

tgcggcaatt cagtggttcg tccaatccat gtcagacccg tctgttgcct tcctaataag 6600

gcacgatcgt accaccttac ttccaccaat cggcatgcac ggtgcttttt ctctccttgt 6660

aaggcatgtt gctaactcat cgttaccatg ttgcaagact acaagagtat tgcataagac 6720

tacattctta agggactatg gttgctgact aattgagatg catgctttgc atacttctgc 6780

ctgctgggga gcctggggac tttccacacc tggttgctga ctaattgaga tgcatgcttt 6840

gcatacttct gcctgctggg gagcctgggg actttccaca ccctaactga cacacattcc 6900

acagctggtt ctttccgcct cagaaggtac ctaaccaagt tcctctttca gaggttattt 6960

caggccacgc gtcttaagct agagtactaa ttcactggcc gtcgttttac aacgtcgtga 7020

ctgggaaaac cctggcgtta cccaacttaa tcgccttgca gcacatcccc ctttcgccag 7080

ctggcgtaat agcgaagagg cccgcaccga tcgcccttcc caacagttgc gcagcctgaa 7140

tggcgaatgg cgcctgatgc ggtattttct ccttacgcat ctgtgcggta tttcacaccg 7200

catatatggt gcactctcag tacaatctgc tctgatgccg catagtaact atcgctacgt 7260

gactgggtca tggctgcgcc ccgacacccg ccaacacccg ctgacgcgcc ctgacgggct 7320

tgtctgctcc cggcatccgc ttacagacaa gctgtgaccg tctccgggag ctgcatgtgt 7380

cagaggtttt caccgtcatc accgaaacgc gcgaggcag 7419

<210> 4
<211> 6909
<212> DNA
<213> Artificial

<220>
<223> vector GT-EDN-SAgp91

<400> 4
cttgaagacg aaagggcctc gtgatacgcc tatttttata ggttaatgtc atgataataa 60

tggtttctta gacgtcaggt ggcacttttc ggggaaatgt gcgcggaacc cctatttgtt 120

tatttttcta aatacattca aatatgtatc cgctcatgag acaataaccc tgataaatgc 180

ttcaataata ttgaaaaagg aagagtatga gtattcaaca tttccgtgtc gcccttattc 240

ccttttttgc ggcattttgc cttcctgttt ttgctcaccc agaaacgctg gtgaaagtaa 300

aagatgctga agatcagttg ggtgcacgag tgggttacat cgaactggat ctcaacagcg 360

gtaagatcct tgagagtttt cgccccgaag aacgttttcc aatgatgagc acttttaaag 420

ttctgctatg tggcgcggta ttatcccgta ttgacgccgg gcaagagcaa ctcggtcgcc 480

gcatacacta ttctcagaat gacttggttg agtactcacc agtcacagaa aagcatctta 540

cggatggcat gacagtaaga gaattatgca gtgctgccat aaccatgagt gataacactg 600

cggccaactt acttctgaca acgatcggag gaccgaagga gctaaccgct tttttgcaca 660

acatggggga tcatgtaact cgccttgatc gttgggaacc ggagctgaat gaagccatac 720

caaacgacga gcgtgacacc acgatgccag cagcaatggc aacaacgttg cgcaaactat 780

taactggcga actacttact ctagcttccc ggcaacaatt aatagactgg atggaggcgg 840

ataaagttgc aggaccactt ctgcgctcgg cccttccggc tggctggttt attgctgata 900

aatctggagc cggtgagcgt gggtctcgcg gtatcattgc agcactgggg ccagatggta 960

agccctcccg tatcgtagtt atctacacga cggggagtca ggcaactatg gatgaacgaa 1020

atagacagat cgctgagata ggtgcctcac tgattaagca ttggtaactg tcagaccaag 1080

tttactcata tatactttag attgatttaa aacttcattt ttaatttaaa aggatctagg 1140

tgaagatcct ttttgataat ctcatgacca aaatccctta acgtgagttt tcgttccact 1200

gagcgtcaga ccccgtagaa aagatcaaag gatcttcttg agatcctttt tttctgcgcg 1260

taatctgctg cttgcaaaca aaaaaaccac cgctaccagc ggtggtttgt ttgccggatc 1320

aagagctacc aactcttttt ccgaaggtaa ctggcttcag cagagcgcag ataccaaata 1380

ctgtccttct agtgtagccg tagttaggcc accacttcaa gaactctgta gcaccgccta 1440

catacctcgc tctgctaatc ctgttaccag tggctgctgc cagtggcgat aagtcgtgtc 1500

ttaccgggtt ggactcaaga cgatagttac cggataaggc gcagcggtcg ggctgaacgg 1560

ggggttcgtg cacacagccc agcttggagc gaacgaccta caccgaactg agatacctac 1620

agcgtgagca ttgagaaagc gccacgcttc ccgaagggag aaaggcggac aggtatccgg 1680

taagcggcag ggtcggaaca ggagagcgca cgagggagct tccaggggga aacgcctggt 1740

atctttatag tcctgtcggg tttcgccacc tctgacttga gcgtcgattt ttgtgatgct 1800

cgtcaggggg gcggagccta tggaaaaacg ccagcaacgc ggccttttta cggttcctgg 1860

ccttttgctg gccttttgct cacatgttct ttcctgcgtt atcccctgat tctgtggata 1920

accgtattac cgcctttgag tgagctgata ccgctcgccg cagccgaacg accgagcgca 1980

gcgagtcagt gagcgaggaa gcggaagagc gcccaatacg caaaccgcct ctccccgcgc 2040

gttggccgat tcattaatgc agctggcacg acaggtttcc cgactggaaa gcgggcagtg 2100

agcgcaacgc aattaatgtg agttagctca ctcattaggc accccaggct ttacacttta 2160

tgcttccggc tcgtatgttg tgtggaattg tgagcggata acaatttcac acaggaaaca 2220

gctatgacca tgattacgcc aaagctctcg agaatgaaag acccccgagg tgggcagtca 2280

atcactcaga ggagaccctc ccaaggaaca gcgagaccac gagtcggatg caactgcaag 2340

agggtttatt gagaacacgg gtacccgggc gactcagtct gtcggaggac tggcgcgccg 2400

agtgaggggt tgtgaattcg atatcgctag cggtggggtc tttcattccc ccctttttct 2460

ggagactaaa taaaatcttt tattctatct atggctcgtg ttaagcttta tcgaattcga 2520

caacaccacg gaattatcag tgcccagcaa cctagcccct gtccagcagc gggcaaggca 2580

ggcggcgatg agttctgccg tggcgatcgg gagggggaaa gcgaaagtcc cagaaaggag 2640

ttgacaggtg gtggcaatgc cccagccagt gggggttgcg tcagcaaaca cagagcacac 2700

cacgccacgt tgacggacaa cgggccacaa ctcctctaaa agagacagca accaggattt 2760

atacaaggag gagaaaacga aagccgtacg ggaagcaata gctagataca gaggctataa 2820

agcagcatat ccacacagcg taaaaggagc aacatagtta agaatatcag tcaatctttc 2880

acaaattttg taatccagag gttgattaac aggaacagag cgtaaataac gggaaagttt 2940

cttaacatgt ttgtcttgtg gcaatacacc tgaactagta attacatatc cctaaaaatg 3000

taaatgattg ccccaccatt ttgttttatt aacatttaaa tgtataccca aatcaagaaa 3060

aacagaacaa atatgggaat aaatggcggt aagatgctct cgcgagggag aagagagctg 3120

acgttagtgc ttaggagacg ttgcacactt tgcagacagg aagtaaagga aatggcgacc 3180

agatctgtag ggcgcagtag tccagggttt ccttgatgat gtcatactta tcctgtccct 3240

tttttttcca cagctcgcgg ttgaggacaa actcttcgcg gtctttccag tggggatcga 3300

cggtatcgat tgattgattg agccgccacc atgggcaact gggccgtgaa cgagggcctg 3360

agcatcttcg tgatcctggt gtggctgggc ctgaacgtgt tcctgttcgt gtggtactac 3420

cgggtgtacg acatcccccc caagttcttc tacacccgga agctgctggg cagcgccctg 3480

gccctggcca gagcccctgc cgcctgcctg aacttcaact gcatgctgat cctgctgccc 3540

gtgtgccgga acctgctgtc cttcctgcgg ggcagcagcg cctgctgcag caccagagtg 3600

cggcggcagc tggaccggaa cctgaccttc cacaagatgg tggcctggat gatcgccctg 3660

cacagcgcca tccacaccat cgcccacctg ttcaacgtgg agtggtgcgt gaacgcccgg 3720

gtgaacaaca gcgaccccta cagcgtggcc ctgagcgagc tgggcgaccg gcagaacgag 3780

agctacctga acttcgcccg gaagcggatc aagaaccccg agggcggcct gtacctggcc 3840

gtgaccctgc tggccggcat caccggcgtg gtgatcaccc tgtgcctgat cctgatcatc 3900

accagcagca ccaagaccat ccggcggagc tacttcgagg tgttctggta cacccaccac 3960

ctgttcgtga tctttttcat cggcctggcc atccacggcg ccgagcggat cgtgaggggc 4020

cagaccgccg agagcctggc cgtgcacaac atcaccgtgt gcgagcagaa aatcagcgag 4080

tggggcaaga tcaaagagtg ccccatcccc cagttcgccg gcaacccccc catgacctgg 4140

aagtggatcg tgggccccat gttcctgtac ctgtgcgagc ggctggtgcg gttctggcgg 4200

agccagcaga aagtggtgat taccaaggtg gtgacccacc ccttcaagac catcgagctg 4260

cagatgaaga aaaagggctt caagatggaa gtgggccagt acatctttgt gaagtgcccc 4320

aaggtgtcca agctggaatg gcaccccttc accctgacca gcgcccctga agaggacttc 4380

ttcagcatcc acatcagaat cgtgggcgac tggaccgagg gcctgttcaa tgcctgcggc 4440

tgcgacaagc aggaattcca ggacgcctgg aagctgccca agatcgccgt ggacggcccc 4500

tttggcaccg ccagcgagga cgtgttcagc tacgaggtgg tgatgctggt cggagccggc 4560

atcggcgtga cccccttcgc cagcatcctg aagagcgtgt ggtacaagta ctgcaacaac 4620

gccaccaacc tgaagctgaa gaagatctac ttctactggc tgtgccggga cacccacgcc 4680

ttcgagtggt tcgccgatct gctgcagctg ctggaaagcc agatgcagga acggaacaac 4740

gccggcttcc tgagctacaa catctacctg accggctggg acgagagcca ggccaaccac 4800

ttcgccgtgc accacgacga ggaaaaggac gtgatcaccg gcctgaagca gaaaaccctg 4860

tacggcaggc ccaactggga caacgagttt aagaccatcg ccagccagca ccccaacacc 4920

cggatcggcg tgtttctgtg cggccctgag gccctggccg agacactgag caagcagagc 4980

atcagcaaca gcgagagcgg ccccaggggc gtgcacttca tcttcaacaa agaaaacttc 5040

tgatgagtat acatttgtat ctagagctcg ctgatcagcc tcgactgtgc cttctagttg 5100

ccagccatct gttgtttgcc cctcccccgt gccttccttg accctggaag gtgccactcc 5160

cactgtcctt tcctaataaa atgaggaaat tgcatcgcat tgtctgagta ggtgtcattc 5220

tattctgggg ggtggggtgg ggcaggacag caagggggag gattgggaag acaatagcag 5280

gcatgctggg gatgcggtgg gctctatggc ttctgagacg gaaagaacca gctggggctc 5340

gatcctctag agcggccgct attttcagac aatacagaac cacgtcagac agagacaaca 5400

cagaacgatg ctgcagcaga caagacgcgc ggcgcggttt cggcgtaaaa ccgaaagcaa 5460

aaattcagac gggggcggga agtgtctctg tctctcgtcc cccaacaaag ccacgtaccc 5520

ctcggacagt ggcggagctc caaaaagtcc gactcgggta ggttaactga tacagaatgg 5580

gccacaaaac gggcccccga ggccgctggg acgtctccca ggggctgcgg ccgggaatac 5640

gaactcgtca gttcttccgc gggaccgcca gatacagatc tagttagcca actagtacag 5700

acgcaggcgc aaacactaga tgccggcaca aacacgccca aagacagaga cagacacgaa 5760

acgaccgctg gccagcttac ctcccggcgg gggggtcggt ggtccctggg caggggtctc 5820

caaatctcgg tggaacctcc aaatgaaaga cccccgaggt gggcagtcaa tcaatctgag 5880

gagaccctcc caaggatcag cgagtccacg attcggatgc aaacagcaag aggctttatt 5940

gggaatacgg gtacccgggc gacgcagtca atcggaggac tggcgcgttt attgtatcga 6000

gctaggcact taaatacaat atctctgcaa tgcggcaatt cagtggttcg tccaatccat 6060

gtcagacccg tctgttgcct tcctaataag gcacgatcgt accaccttac ttccaccaat 6120

cggcatgcac ggtgcttttt ctctccttgt aaggcatgtt gctaactcat cgttaccatg 6180

ttgcaagact acaagagtat tgcataagac tacattctta agggactatg gttgctgact 6240

aattgagatg catgctttgc atacttctgc ctgctgggga gcctggggac tttccacacc 6300

tggttgctga ctaattgaga tgcatgcttt gcatacttct gcctgctggg gagcctgggg 6360

actttccaca ccctaactga cacacattcc acagctggtt ctttccgcct cagaaggtac 6420

ctaaccaagt tcctctttca gaggttattt caggccacgc gtcttaagct agagtactaa 6480

ttcactggcc gtcgttttac aacgtcgtga ctgggaaaac cctggcgtta cccaacttaa 6540

tcgccttgca gcacatcccc ctttcgccag ctggcgtaat agcgaagagg cccgcaccga 6600

tcgcccttcc caacagttgc gcagcctgaa tggcgaatgg cgcctgatgc ggtattttct 6660

ccttacgcat ctgtgcggta tttcacaccg catatatggt gcactctcag tacaatctgc 6720

tctgatgccg catagttaagt atcgctacgt gactgggtca tggctgcgcc ccgacacccg 6780

ccaacacccg ctgacgcgcc ctgacgggct tgtctgctcc cggcatccgc ttacagacaa 6840

gctgtgaccg tctccgggag ctgcatgtgt cagaggtttt caccgtcatc accgaaacgc 6900

gcgaggcag 6909

<210> 5
<211> 7340
<212> DNA
<213> Artificial

<220>
<223> vector GT-T2Agp91IRESSD

<400> 5
cttgaagacg aaagggcctc gtgatacgcc tatttttata ggttaatgtc atgataataa 60

tggtttctta gacgtcaggt ggcacttttc ggggaaatgt gcgcggaacc cctatttgtt 120

tatttttcta aatacattca aatatgtatc cgctcatgag acaataaccc tgataaatgc 180

ttcaataata ttgaaaaagg aagagtatga gtattcaaca tttccgtgtc gcccttattc 240

ccttttttgc ggcattttgc cttcctgttt ttgctcaccc agaaacgctg gtgaaagtaa 300

aagatgctga agatcagttg ggtgcacgag tgggttacat cgaactggat ctcaacagcg 360

gtaagatcct tgagagtttt cgccccgaag aacgttttcc aatgatgagc acttttaaag 420

ttctgctatg tggcgcggta ttatcccgta ttgacgccgg gcaagagcaa ctcggtcgcc 480

gcatacacta ttctcagaat gacttggttg agtactcacc agtcacagaa aagcatctta 540

cggatggcat gacagtaaga gaattatgca gtgctgccat aaccatgagt gataacactg 600

cggccaactt acttctgaca acgatcggag gaccgaagga gctaaccgct tttttgcaca 660

acatggggga tcatgtaact cgccttgatc gttgggaacc ggagctgaat gaagccatac 720

caaacgacga gcgtgacacc acgatgccag cagcaatggc aacaacgttg cgcaaactat 780

taactggcga actacttact ctagcttccc ggcaacaatt aatagactgg atggaggcgg 840

ataaagttgc aggaccactt ctgcgctcgg cccttccggc tggctggttt attgctgata 900

aatctggagc cggtgagcgt gggtctcgcg gtatcattgc agcactgggg ccagatggta 960

agccctcccg tatcgtagtt atctacacga cggggagtca ggcaactatg gatgaacgaa 1020

atagacagat cgctgagata ggtgcctcac tgattaagca ttggtaactg tcagaccaag 1080

tttactcata tatactttag attgatttaa aacttcattt ttaatttaaa aggatctagg 1140

tgaagatcct ttttgataat ctcatgacca aaatccctta acgtgagttt tcgttccact 1200

gagcgtcaga ccccgtagaa aagatcaaag gatcttcttg agatcctttt tttctgcgcg 1260

taatctgctg cttgcaaaca aaaaaaccac cgctaccagc ggtggtttgt ttgccggatc 1320

aagagctacc aactcttttt ccgaaggtaa ctggcttcag cagagcgcag ataccaaata 1380

ctgtccttct agtgtagccg tagttaggcc accacttcaa gaactctgta gcaccgccta 1440

catacctcgc tctgctaatc ctgttaccag tggctgctgc cagtggcgat aagtcgtgtc 1500

ttaccgggtt ggactcaaga cgatagttac cggataaggc gcagcggtcg ggctgaacgg 1560

ggggttcgtg cacacagccc agcttggagc gaacgaccta caccgaactg agatacctac 1620

agcgtgagca ttgagaaagc gccacgcttc ccgaagggag aaaggcggac aggtatccgg 1680

taagcggcag ggtcggaaca ggagagcgca cgagggagct tccaggggga aacgcctggt 1740

atctttatag tcctgtcggg tttcgccacc tctgacttga gcgtcgattt ttgtgatgct 1800

cgtcaggggg gcggagccta tggaaaaacg ccagcaacgc ggccttttta cggttcctgg 1860

ccttttgctg gccttttgct cacatgttct ttcctgcgtt atcccctgat tctgtggata 1920

accgtattac cgcctttgag tgagctgata ccgctcgccg cagccgaacg accgagcgca 1980

gcgagtcagt gagcgaggaa gcggaagagc gcccaatacg caaaccgcct ctccccgcgc 2040

gttggccgat tcattaatgc agctggcacg acaggtttcc cgactggaaa gcgggcagtg 2100

agcgcaacgc aattaatgtg agttagctca ctcattaggc accccaggct ttacacttta 2160

tgcttccggc tcgtatgttg tgtggaattg tgagcggata acaatttcac acaggaaaca 2220

gctatgacca tgattacgcc aaagctctcg agaatgaaag acccccgagg tgggcagtca 2280

atcactcaga ggagaccctc ccaaggaaca gcgagaccac gagtcggatg caactgcaag 2340

agggtttatt gagaacacgg gtacccgggc gactcagtct gtcggaggac tggcgcgccg 2400

agtgaggggt tgtgaattcg atatcgctag cggtggggtc tttcattccc ccctttttct 2460

ggagactaaa taaaatcttt tattctatct atggctcgtg ttaagcttta tcgaattcga 2520

caacaccacg gaattatcag tgcccagcaa cctagcccct gtccagcagc gggcaaggca 2580

ggcggcgatg agttctgccg tggcgatcgg gagggggaaa gcgaaagtcc cagaaaggag 2640

ttgacaggtg gtggcaatgc cccagccagt gggggttgcg tcagcaaaca cagagcacac 2700

cacgccacgt tgacggacaa cgggccacaa ctcctctaaa agagacagca accaggattt 2760

atacaaggag gagaaaacga aagccgtacg ggaagcaata gctagataca gaggctataa 2820

agcagcatat ccacacagcg taaaaggagc aacatagtta agaatatcag tcaatctttc 2880

acaaattttg taatccagag gttgattaac aggaacagag cgtaaataac gggaaagttt 2940

cttaacatgt ttgtcttgtg gcaatacacc tgaactagta attacatatc cctaaaaatg 3000

taaatgattg ccccaccatt ttgttttatt aacatttaaa tgtataccca aatcaagaaa 3060

aacagaacaa atatgggaat aaatggcggt aagatgctct cgcgaccaga tctgtagggc 3120

gcagtagtcc agggtttcct tgatgatgtc atacttatcc tgtccctttt ttttccacag 3180

ctcgcggttg aggacaaact cttcgcggtc tttccagtgg ggatcgacgg tatcgataag 3240

cttgatctcc gagggcagag gaagtcttct aacatgcggt gacgtggagg agaatcccgg 3300

ccctggcaac tgggccgtga acgagggcct gagcatcttc gtgatcctgg tgtggctggg 3360

cctgaacgtg ttcctgttcg tgtggtacta ccgggtgtac gacatccccc ccaagttctt 3420

ctacacccgg aagctgctgg gcagcgccct ggccctggcc agagcccctg ccgcctgcct 3480

gaacttcaac tgcatgctga tcctgctgcc cgtgtgccgg aacctgctgt ccttcctgcg 3540

gggcagcagc gcctgctgca gcaccagagt gcggcggcag ctggaccgga acctgacctt 3600

ccacaagatg gtggcctgga tgatcgccct gcacagcgcc atccacacca tcgcccacct 3660

gttcaacgtg gagtggtgcg tgaacgcccg ggtgaacaac agcgacccct acagcgtggc 3720

cctgagcgag ctgggcgacc ggcagaacga gagctacctg aacttcgccc ggaagcggat 3780

caagaacccc gagggcggcc tgtacctggc cgtgaccctg ctggccggca tcaccggcgt 3840

ggtgatcacc ctgtgcctga tcctgatcat caccagcagc accaagacca tccggcggag 3900

ctacttcgag gtgttctggt acacccacca cctgttcgtg atctttttca tcggcctggc 3960

catccacggc gccgagcgga tcgtgagggg ccagaccgcc gagagcctgg ccgtgcacaa 4020

catcaccgtg tgcgagcaga aaatcagcga gtggggcaag atcaaagagt gccccatccc 4080

ccagttcgcc ggcaaccccc ccatgacctg gaagtggatc gtgggcccca tgttcctgta 4140

cctgtgcgag cggctggtgc ggttctggcg gagccagcag aaagtggtga ttaccaaggt 4200

ggtgacccac cccttcaaga ccatcgagct gcagatgaag aaaaagggct tcaagatgga 4260

agtgggccag tacatctttg tgaagtgccc caaggtgtcc aagctggaat ggcacccctt 4320

caccctgacc agcgcccctg aagaggactt cttcagcatc cacatcagaa tcgtgggcga 4380

ctggaccgag ggcctgttca atgcctgcgg ctgcgacaag caggaattcc aggacgcctg 4440

gaagctgccc aagatcgccg tggacggccc ctttggcacc gccagcgagg acgtgttcag 4500

ctacgaggtg gtgatgctgg tcggagccgg catcggcgtg acccccttcg ccagcatcct 4560

gaagagcgtg tggtacaagt actgcaacaa cgccaccaac ctgaagctga agaagatcta 4620

cttctactgg ctgtgccggg acacccacgc cttcgagtgg ttcgccgatc tgctgcagct 4680

gctggaaagc cagatgcagg aacggaacaa cgccggcttc ctgagctaca acatctacct 4740

gaccggctgg gacgagagcc aggccaacca cttcgccgtg caccacgacg aggaaaagga 4800

cgtgatcacc ggcctgaagc agaaaaccct gtacggcagg cccaactggg acaacgagtt 4860

taagaccatc gccagccagc accccaacac ccggatcggc gtgtttctgt gcggccctga 4920

ggccctggcc gagacactga gcaagcagag catcagcaac agcgagagcg gccccagggg 4980

cgtgcacttc atcttcaaca aagaaaactt ctgatgagta tacatttgta tctagacgcc 5040

cctctccctc ccccccccct aacgttactg gccgaagccg cttggaataa ggccggtgtg 5100

cgtttgtcta tatgttattt tccaccatat tgccgtcttt tggcaatgtg agggcccgga 5160

aacctggccc tgtcttcttg acgagcattc ctaggggtct ttcccctctc gccaaaggaa 5220

tgcaaggtct gttgaatgtc gtgaaggaag cagttcctct ggaagcttct tgaagacaaa 5280

caacgtctgt agcgaccctt tgcaggcagc ggaacccccc acctggcgac aggtgcctct 5340

gcggccaaaa gccacgtgta taagatacac ctgcaaaggc ggcacaaccc cagtgccacg 5400

ttgtgagttg gatagttgtg gaaagagtca aatggctctc ctcaagcgta ttcaacaagg 5460

ggctgaagga tgcccagaag gtaccccatt gtatgggatc tgatctgggg cctcggtgca 5520

catgctttac atgtgtttag tcgaggttaa aaaacgtcta ggccccccga accacgggga 5580

cgtggttttc ctttgaaaaa cacgatgata atcgtcctca ctctctaccg catcgctgtc 5640

tgcgagggcc agctgttggg gtgagtactc cctctcaaaa gcgggcatga cttctgcgct 5700

aagattgtca gtttccaaaa acgaggagga tttgatattc acctggcccg cggtgatgcc 5760

tttgagggtg gccgcgtcta gagcggccgc tattttcaga caatacagaa ccacgtcaga 5820

cagagacaac acagaacgat gctgcagcag acaagacgcg cggcgcggtt tcggcgtaaa 5880

accgaaagca aaaattcaga cgggggcggg aagtgtctct gtctctcgtc ccccaacaaa 5940

gccacgtacc cctcggacag tggcggagct ccaaaaagtc cgactcgggt aggttaactg 6000

atacagaatg ggccacaaaa cgggcccccg aggccgctgg gacgtctccc aggggctgcg 6060

gccgggaata cgaactcgtc agttcttccg cgggaccgcc agatacagat ctagttagcc 6120

aactagtaca gacgcaggcg caaacactag atgccggcac aaacacgccc aaagacagag 6180

acagacacga aacgaccgct ggccagctta cctcccggcg ggggggtcgg tggtccctgg 6240

gcaggggtct ccaaatctcg gtggaacctc caaatgaaag acccccgagg tgggcagtca 6300

atcaatctga ggagaccctc ccaaggatca gcgagtccac gattcggatg caaacagcaa 6360

gaggctttat tgggaatacg ggtacccggg cgacgcagtc aatcggagga ctggcgcgtt 6420

tattgtatcg agctaggcac ttaaatacaa tatctctgca atgcggcaat tcagtggttc 6480

gtccaatcca tgtcagaccc gtctgttgcc ttcctaataa ggcacgatcg taccacctta 6540

cttccaccaa tcggcatgca cggtgctttt tctctccttg taaggcatgt tgctaactca 6600

tcgttaccat gttgcaagac tacaagagta ttgcataaga ctacattctt aagggactat 6660

ggttgctgac taattgagat gcatgctttg catacttctg cctgctgggg agcctgggga 6720

ctttccacac ctggttgctg actaattgag atgcatgctt tgcatacttc tgcctgctgg 6780

ggagcctggg gactttccac accctaactg acacacattc cacagctggt tctttccgcc 6840

tcagaaggta cctaaccaag ttcctctttc agaggttatt tcaggccacg cgtcttaagc 6900

tagagtacta attcactggc cgtcgtttta caacgtcgtg actgggaaaa ccctggcgtt 6960

acccaactta atcgccttgc agcacatccc cctttcgcca gctggcgtaa tagcgaagag 7020

gcccgcaccg atcgcccttc ccaacagttg cgcagcctga atggcgaatg gcgcctgatg 7080

cggtattttc tccttacgca tctgtgcggt atttcacacc gcatatatgg tgcactctca 7140

gtacaatctg ctctgatgcc gcatagtaac tatcgctacg tgactgggtc atggctgcgc 7200

cccgacaccc gccaacaccc gctgacgcgc cctgacgggc ttgtctgctc ccggcatccg 7260

cttacagaca agctgtgacc gtctccggga gctgcatgtg tcagaggttt tcaccgtcat 7320

caccgaaacg cgcgaggcag 7340